# EUROPEAN PATENT SPECIFICATION

(11) **EP 3 160 489 B1**
(45) Date of publication and mention of the grant of the patent: **07.06.2023**
(21) Application number: 15732533.3
(22) Date of filing: 26.06.2015
(51) Int. Cl.: A61K 38/17, C07K 14/47, A61K 48/00, C07K 16/40, C12N 15/00, A61P 25/28, A61P 3/10, A61P 41/00, A61P 13/12, A61P 1/02, A61P 3/04, A61P 7/00, A61P 7/04, A61P 7/06, A61P 9/12, A61P 9/14, A61P 17/00, A61P 17/06, A61P 17/10, A61P 19/08

(54) **CELL-PERMEABLE PEPTIDE INHIBITORS OF THE JNK SIGNAL TRANSDUCTION PATHWAY FOR THE TREATMENT OF CYSTITIS**
ZELLDURCHLÄSSIGEN PEPTIDHEMMERN DES JNK-SIGNALTRANSDUKTIONSWEGES ZUR BEHANDLUNG VON ZYSTITIS
INHIBITEURS PEPTIDIQUES PERMÉABLES AUX CELLULES DE LA VOIE DE TRANSDUCTION DU SIGNAL JNK POUR LE TRAITEMENT DE LA CYSTITE

(30) Priority: 26.06.2014 WO PCT/EP2014/001736; 08.10.2014 WO PCT/EP2014/002724
(43) Date of publication of application: 03.05.2017
(73) Proprietor: Xigen Inflammation Ltd., 3030 Limassol (CY)
(72) Inventor: COMBETTE, Jean-Marc, F-74140 Saint Cergues (FR); DELOCHE, Catherine, CH-1207 Genf (CH)
(74) Representative: Weiss, Wolfgang
(86) International application number: PCT/EP2015/001294
(87) International publication number: WO 2015/197194

(56) References cited:
- EP-B1- 2 627 346
- WO-A1-98/49188
- WO-A1-2009/143865
- WO-A1-2010/091310
- WO-A1-2012/048893
- WO-A1-2012/048893
- WO-A1-2013/091670
- WO-A1-2013/091896
- WO-A1-2014/206426
- WO-A2-2004/037196
- WO-A2-2010/065850
- US-A1- 2005 148 624
- US-A1- 2006 094 753
- US-A1- 2006 094 753
- US-A1- 2006 172 991
- US-A1- 2012 101 046
- US-A1- 2012 101 046
- US-A1- 2012 101 046
- US-A1- 2013 337 557
- US-A1- 2013 337 557
- US-A1- 2017 128 516
- DUGAN C ET AL: "Role of c-Jun N-terminal kinase (JNK) activation in micturition reflexes in cyclophosphamide (CYP)-induced cystitis in female rats", JOURNAL OF MOLECULAR NEUROSCIENCE, BIRKHAEUSER, CAMBRIDGE, MA, US, vol. 54, no. 3, 1 November 2014 (2014-11-01), pages 360-369, XP002752705, ISSN: 0895-8696, DOI: 10.1007/S12031-014-0308-5 [retrieved on 2014-04-26]

## Description

The present invention refers to protein kinase inhibitors and more specifically to the inhibitors of the protein kinase c-Jun amino terminal kinase, chimeric peptides for use in the treatment of various novel diseases or disorders strongly related to JNK signaling, wherein the disease or disorder is cystitis.

The c-Jun amino terminal kinase (JNK) is a member of the stress-activated group of mitogen-activated protein (MAP) kinases. These kinases have been implicated in the control of cell growth and differentiation, and, more generally, in the response of cells to environmental stimuli. The JNK signal transduction pathway is activated in response to environmental stress and by the engagement of several classes of cell surface receptors. These receptors can include cytokine receptors, serpentine receptors and receptor tyrosine kinases. In mammalian cells, JNK has been implicated in biological processes such as oncogenic transformation and mediating adaptive responses to environmental stress. JNK has also been associated with modulating immune responses, including maturation and differentiation of immune cells, as well as effecting programmed cell death in cells identified for destruction by the immune system. This unique property makes JNK signaling a promising target for developing pharmacological intervention. Among several neurological disorders, JNK signaling is particularly implicated in ischemic stroke and Parkinson's disease, but also in other diseases as mentioned further below. Furthermore, the mitogen-activated protein kinase (MAPK) p₃₈alpha was shown to negatively regulate the cell proliferation by antagonizing the JNK-cJun-pathway. The mitogen-activated protein kinase (MAPK) p₃₈alpha therefore appears to be active in suppression of normal and cancer cell proliferation and, as a further, demonstrates the involvement of JNK in cancer diseases (see e.g. Hui et al., Nature Genetics, Vol 39, No. 6, June 2007). It was also shown, that c-Jun N-terminal Kinase (JNK) is involved in neuropathic pain produced by spinal nerve ligation (SNL), wherein SNL induced a slow and persistent activation of JNK, in particular JNK1, wheras p38 mitogen-activated protein kinase activation was found in spinal microglia after SNL, which had fallen to near basal lavel by 21 days (Zhuang et al., The Journal of Neuroscience, March 29, 2006, 26(13):3551-3560)).

Inhibition or interruption of JNK signaling pathway, particularly the provision of inhibitors of the JNK signaling pathway, thus appears to be a promising approach in combating disorders strongly related to JNK signaling. However, there are only a few inhibitors of the JNK signaling pathway known so far.

Inhibitors of the JNK signaling pathway as already known in the prior art, particularly include e.g. upstream kinase inhibitors (for example, CEP-1347), small chemical inhibitors of JNK (SP600125 and AS601245), which directly affect kinase activity e.g. by competing with the ATP-binding site of the protein kinase, and peptide inhibitors of the interaction between JNK and its substrates (D-JNKI and I-JIP) (see e.g. Kuan et al., Current Drug Targets - CNS & Neurological Disorders, February 2005, vol. 4, no. 1, pp. 63-67(5)).

The upstream kinase inhibitor CEP-1347 (KT7515) is a semisynthetic inhibitor of the mixed lineage kinase family. CEP-1347 (KT7515) promotes neuronal survival at dosages that inhibit activation of the c-Jun amino-terminal kinases (JNKs) in primary embryonic cultures and differentiated PC12 cells after trophic withdrawal and in mice treated with 1-methyl-4-phenyl tetrahydropyridine. Further, CEP-1347 (KT7515) can promote long term-survival of cultured chick embryonic dorsal root ganglion, sympathetic, ciliary and motor neurons (see e.g. Borasio et al., Neuroreport. 9(7): 1435-1439, May 11th 1998.).

The small chemical JNK inhibitor SP600125 was found to reduce the levels of c-Jun phosphorylation, to protect dopaminergic neurons from apoptosis, and to partly restore the level of dopamine in MPTP-induced PD in C57BL/6N mice (Wang et al., Neurosci Res. 2004 Feb; 48(2); 195-202). These results furthermore indicate that JNK pathway is the major mediator of the neurotoxic effects of MPTP *in vivo* and inhibiting JNK activity may represent a new and effective strategy to treat PD.

A further example of small chemical inhibitors is the aforementioned JNK-Inhibitor AS601245. AS601245 inhibits the JNK signalling pathway and promotes cell survival after cerebral ischemia. *In vivo,* AS601245 provided significant protection against the delayed loss of hippocampal CA1 neurons in a gerbil model of transient global ischemia. This effect is mediated by JNK inhibition and therefore by c-Jun expression and phosphorylation (see e.g. Carboni et al., J Pharmacol Exp Ther. 2004 Jul; 310(1):25-32. Epub 2004 Feb 26^{th}).

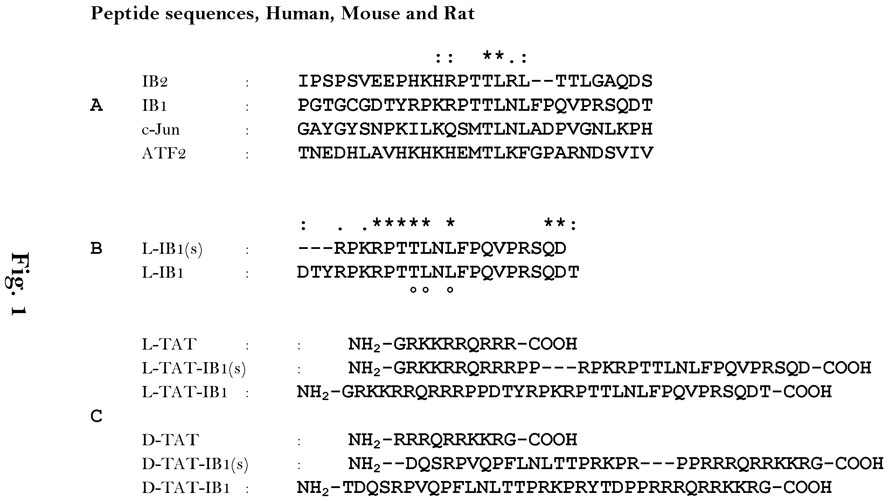

A third class of inhibitors of the JNK signaling pathway represent peptide inhibitors of the interaction between JNK and its substrates, as mentioned above. As a starting point for construction of such JNK inhibitor peptides a sequence alignment of naturally occurring JNK proteins may be used. Typically, these proteins comprise JNK binding domains (JBDs) and occur in various insulin binding (IB) proteins, such as IB1 or IB2. The results of such an exemplary sequence alignment is e.g. a sequence alignment between the JNK binding domains of IB1 [SEQ ID NO: 13], IB2 [SEQ ID NO: 14], c-Jun [SEQ ID NO: 15] and ATF2 [SEQ ID NO: 16] (see e.g. FIGS. 1A-1C). Such an alignment reveals a partially conserved 8 amino acid sequence (see e.g. Figure 1A). A comparison of the JBDs of IB1 and IB2 further reveals two blocks of seven and three amino acids that are highly conserved between the two sequences.

Sequences constructed on basis of such an alignment are e.g. disclosed in WO 01/27268 or in WO 2007/031280. WO 2007/031280 and WO 01/27268 disclose small cell permeable fusion peptides, comprising a so-called TAT cell permeation sequence derived from the basic trafficking sequence of the HIV-TAT protein and a minimum 20 amino acid inhibitory sequence of IB1. Both components are covalently linked to each other. Exemplary (and at present the only) inhibitors of the MAPK-JNK signaling pathway disclosed in both WO 2007/031280 and WO 01/27268, are e.g. L-JNKI1 (JNK-inhibitor peptide composed of L amino acids) or the protease resistant D-JNKI1 peptides (JNK-inhibitor peptide composed of non-native D amino acids). These JNK-inhibitor (JNKI) peptides are specific for JNK (JNK1, JNK2 and JNK3). In contrast to those small compound inhibitors as discussed above, the inhibitor sequences in WO 2007/031280 or WO 01/27268, e.g. JNKI1, rather inhibit the interaction between JNK and its substrate. By its trafficking sequence derived from TAT, the fusion peptide is efficiently transported into cells. Due to the novel properties obtained by the trafficking component the fusion peptides are actively transported into cells, where they remain effective until proteolytic degradation.

However, peptides according to WO 2007/031280 or WO 01/27268 have only shown to be active in a particularly limited number of diseases, particularly non-malignant or immunological-related cell proliferative diseases.

One object of the present invention is thus, to identify further diseases, which can be combated with JNK inhibitor peptides. Another object of the present invention is to provide new JNK inhibitor peptides and derivatives thereof for use in the treatment and/or prevention of those diseases and of diseases not yet or already known to be strongly related to JNK signaling.

This object is solved by a JNK inhibitor chimeric peptide comprising at least one first domain and at least one second domain linked by a covalent bond, the first domain comprising a trafficking sequence, and the second domain comprising a JNK inhibitor sequence as defined in SEQ ID NO: 11, for use in treating and/or preventing various inflammatory or non-inflammatory diseases strongly related to JNK signaling in a subject by intravesical administration, wherein the diseases or disorders are selected from:
cystitis in general, in particular interstitial cystitis.
C-Jun N-terminal kinases (JNKs) are serine-threonine protein kinases, coded by three genes JNK1, JNK2, and JNK3, expressed as ten different isoforms by mRNA alternative splicing, each isoforms being expressed as a short form (46 kDa) and a long form (54 kDa) (Davis, 2000, Cell 103: 239-52). While JNK1 and JNK2 are ubiquitous, JNK3 is mainly expressed in the brain (Kyriakis and Avruch, 2001, Physiol Rev 81: 807-69). JNKs are activated by phosphorylation (pJNK) through MAPKinase activation by extracellular stimuli, such as ultraviolet stress, cytokines and AB peptides and they have multiple functions including gene expression regulation, cell proliferation and apoptosis (Dhanasekaran and Reddy, 2008, Oncogene 27: 6245-51).

The present invention is suitable for use in the treatment of diseases resulting in loss of bladder function. Such diseases are selected from cystitis in general, in particular interstitial cystitis.

Typically, a JNK inhibitor sequence as defined above may be derived from a human or rat IB1 sequence, preferably from an amino acid sequence as defined or encoded by any of sequences according to SEQ ID NO: 102 (depicts the IB1 cDNA sequence from rat and its predicted amino acid sequence), SEQ ID NO: 103 (depicts the IB1 protein sequence from rat encoded by the exon-intron boundary of the RIB1 gene - splice donor), SEQ ID NO: 104 (depicts the IB1 protein sequence from *Homo sapiens),* or SEQ ID NO: 105 (depicts the IB1 cDNA sequence from *Homo sapiens),* more preferably from an amino acid sequence as defined or encoded by any of sequences according to SEQ ID NO: 104 (depicts the IB1 protein sequence from *Homo sapiens*), or SEQ ID NO: 105 (depicts the IB1 cDNA sequence from *Homo sapiens),* or from any fragments or variants thereof. In other words, the JNK inhibitor sequence comprises a fragment, variant, or variant of such fragment of a human or rat IB1 sequence. Human or rat IB sequences are defined or encoded, respectively, by the sequences according to SEQ ID NO: 102, SEQ ID NO: 103, SEQ ID NO: 104 or SEQ ID NO: 105.

According to a particular embodiment, a JNK inhibitor sequence as used herein typically binds JNK and/or inhibits the activation of at least one JNK activated transcription factor, e.g. c-Jun or ATF2 (see e.g. SEQ ID NOs: 15 and 16, respectively) or Elk1.

The JNK inhibitor sequence as used herein comprises one amino acid sequence according to SEQ ID NO: 2.

The JNK inhibitor sequences as used herein is composed in part or exclusively of D-amino acids as defined above. More preferably, these JNK inhibitor sequences composed of D-amino acids are non-native D retro-inverso sequences of the above (native) JNK inhibitor sequences. The term "retro-inverso sequences" refers to an isomer of a linear peptide sequence in which the direction of the sequence is reversed and the chirality of each amino acid residue is inverted (see e.g. Jameson et al., Nature, 368,744-746 (1994); Brady et al., Nature, 368, 692-693 (1994)). The advantage of combining D-enantiomers and reverse synthesis is that the positions of carbonyl and amino groups in each amide bond are exchanged, while the position of the side-chain groups at each alpha carbon is preserved. Unless specifically stated otherwise, it is presumed that any given L-amino acid sequence or peptide described may be converted into an D retro-inverso sequence or peptide by synthesizing a reverse of the sequence or peptide for the corresponding native L-amino acid sequence or peptide.

The D retro-inverso sequences as used herein and as defined above have a variety of useful properties. For example, D retro-inverso sequences as used herein enter cells as efficiently as L-amino acid sequences as used herein, whereas the D retro-inverso sequences as used herein are more stable than the corresponding L-amino acid sequences.

Accordingly, the JNK inhibitor sequences as used herein comprise one D retro-inverso sequence according to the amino acid sequence NH₂-DQSRPVQPFLNLTTPRKPR-COOH (D-IB1(s)) [SEQ ID NO: 2].

The JNK inhibitor sequence as used herein (SEQ ID NO: 2) and the JNK inhibitor sequences as disclosed above (SEQ ID NOs: 1, 3-4, 13-20 and 33-100) are presented in Table 1. The table presents the name of the JNK inhibitor sequences, as well as their sequence identifier number, their length, and amino acid sequence. Furthermore, Table 1 shows sequences as well as their generic formulas, e.g. for SEQ ID NO's: 1, 2, 5, 6, 9 and 11 (according to the invention) and SEQ ID NO's: 3, 4, 7, 8, 10 and 12, respectively. Table 1 furthermore discloses the chimeric sequences SEQ ID NOs: 9-12 and 23-32 (see below), L-IB1 sequences SEQ ID NOs: 33 to 66 and D-IB1 sequences SEQ ID NOs: 67 to 100.

**TABLE 1**

| **SEQUENCE/PEPTIDE NAME** | **SEQ ID NO** | **AA** | **SEQUENCE** |
|---|---|---|---|
| L-IBl(s) | 1 | 19 | RPKRPTTLNLFPQVPRSQD (NH₂-RPKRPTTLNLFPQVPRSQD-COOH) |
| D-IBi(s) | 2 | 19 | DQSRPVQPFLNLTTPRKPR (NH₂-DQSRPVQPFLNLTTPRKPR-COOH) |
| L-IB (generic) (s) | 3 | 19 | NH₂-Xₙ^{b}-Xₙ^{a}-RPTTLXLXXXXXXXQD-Xₙ^{b}-COOH |
| D-IB (generic) (s) | 4 | 19 | NH₂-Xₙ^{b}-DQXXXXXXXLXLTTPR-Xₙ^{a}-Xₙ^{b}-COOH |
| L-TAT | 5 | 10 | GRKKRRQRRR (NH₂-GRKKRRQRRR-COOH) |
| D-TAT | 6 | 10 | RRRQRRKKRG (NH₂-RRRQRRKKRG-COOH) |
| L-generic-TAT (s) | 7 | 11 | NH₂-Xₙ^{b}-RKKRRQRRR-Xₙ^{b}-COOH |
| D-generic-TAT (s) | 8 | 11 | NH₂-Xₙ^{b}-RRRQRRKKR-Xₙ^{b}-COOH |
| L-TAT-IB1(s) | 9 | 31 | GRKKRRQRRRPPRPKRPTTLNLFPQVPRSQD (NH₂-GRKKRRQRRRPPRPKRPTTLNLFPQVPRSQD-COOH) |
| L-TAT-IB (generic) (s) | 10 | 29 | NH₂-Xₙ^{b}-RKKRRQRRR-Xₙ^{b}-xₙ^{a}-RPTTLXLXXXXXXXQD-Xₙ^{b}-COOH |
| D-TAT-IB1(s) | 11 | 31 | DQSRPVQPFLNLTTPRKPRPPRRRQRRKKRG (NH₂-DQSRPVQPFLNLTTPRKPRPPRRRQRRKKRG-COOH) |
| D-TAT-IB (generic) (s) | 12 | 29 | NH₂-Xₙ^{b}-DQXXXXXXXLXLTTPR-Xₙ^{a}-Xₙ^{b}-RRRQRRKKR-Xₙ^{b}-COOH |
| IB1-long | 13 | 29 | PGTGCGDTYRPKRPTTLNLFPQVPRSQDT (NH₂- PGTGCGDTYRPKRPTTLNLFPQVPRSQDT -COOH) |
| IB2-long | 14 | 27 | IPSPSVEEPHKHRPTTLRLTTLGAQDS (NH₂- IPSPSVEEPHKHRPTTLRLTTLGAQDS -COOH) |
| c-Jun | 15 | 29 | GAYGYSNPKILKQSMTLNLADPVGNLKPH (NH₂- GAYGYSNPKILKQSMTLNLADPVGNLKPH -COOH) |
| ATF2 | 16 | 29 | TNEDHLAVHKHKHEMTLKFGPARNDSVIV (NH₂- TNEDHLAVHKHKHEMTLKFGPARNDSVIV -COOH) |
| L-IB1 | 17 | 23 | DTYRPKRPTTLNLFPQVPRSQDT (NH₂- DTYRPKRPTTLNLFPQVPRSQDT -COOH) |
| D-IB 1 | 18 | 23 | TDQSRPVQPFLNLTTPRKPRYTD (NH₂- TDQSRPVQPFLNLTTPRKPRYTD -COOH) |
| L-IB (generic) | 19 | 19 | XRPTTXLXXXXXXXQDS/TX (NH₂- XRPTTLXLXXXXXXXQDS/TX -COOH) |
| D-IB (generic) | 20 | 19 | XS/TDQXXXXXXXLXLTTPRX (NH₂- XS/TDQXXXXXXXLXLTTPRX -COOH) |
| L-generic-TAT | 21 | 17 | XXXXRKKRRQRRRXXXX (NH₂- XXXXRKKRRQRRRXXXX -COOH) |
| D-generic-TAT | 22 | 17 | XXXXRRRQRRKKRXXXX (NH₂- XXXXRRRQRRKKRXXXX -COOH) |
| L-TAT-IB 1 | 23 | 35 | GRKKRRQRRRPPDTYRPKRPTTLNLFPQVPRSQDT (NH₂- GRKKRRQRRRPPDTYRPKRPTTLNLFPQVPRSQDT -COOH) |
| L-TAT-IB (generic) | 24 | 42 | XXXXXXXRKKRRQRRRXXXXXXXXRPTTLXLXXXXXXXODS/TX (NH₂-XXXXXXXRKKRRQRRRXXXXXXXXRPTTLXLXXXXXXXQDS/TX -COOH) |
| D-TAT-IB 1 | 25 | 35 | TDQSRPVQPFLNLTTPRKPRYTDPPRRRQRRKKRG (NH₂- TDQSRPVQPFLNLTTPRKPRYTDPPRRRQRRKKRG -COOH) |
| D-TAT-IB (generic) | 26 | 42 | XT/SDQXXXXXXXLXLTTPRXXXXXXXXRRRQRRKKRXXXXXXX (NH₂-XT/SDQXXXXXXXLXLTTPRXXXXXXXXRRRQRRKKRXXXXXXX -COOH) |
| L-TAT-IB1(s1) | 27 | 30 | RKKRRQRRRPPRPKRPTTLNLFPQVPRSQD (NH₂-RKKRRQRRRPPRPKRPTTLNLFPQVPRSQD-COOH) |
| L-TAT-IB1(s2) | 28 | 30 | GRKKRRQRRRXₙ^{c}RPKRPTTLNLFPQVPRSQD (NH₂-GRKKRRQRRRXₙ^{c}RPKRPTTLNLFPQVPRSQD-COOH) |
| L-TAT-IB1(s3) | 29 | 29 | RKKRRQRRRXₙ^{c}RPKRPTTLNLFPQVPRSQD |
| | | | (NH₂-RKKRRQRRRXₙ^{c}RPKRPTTLNLFPQVPRSQD-COOH) |
| D-TAT-IB1(S1) | 30 | 30 | DQSRPVQPFLNLTTPRKPRPPRRRQRRKKR (NH₂-DQSRPVQPFLNLTTPRKPRPPRRRQRRKKR-COOH) |
| D-TAT-IB1(s2) | 31 | 30 | DQSRPVQPFLNLTTPRKPRXₙ^{c}RRRQRRKKRG (NH₂-DQSRPVQPFLNLTTPRKPRXₙ^{c}RRRQRRKKRG-COOH) |
| D-TAT-IB1(s3) | 32 | 29 | DQSRPVQPFLNLTTPRKPRXₙ^{c}RRRQRRKKR (NH₂-DQSRPVQPFLNLTTPRKPRXₙ^{c}RRRQRRKKR-COOH) |
| L-IB1(s1) | 33 | 13 | TLNLFPQVPRSQD (NH₂-TLNLFPQVPRSQD-COOH) |
| L-IB1(s2) | 34 | 13 | TTLNLFPQVPRSQ (NH₂-TTLNLFPQVPRSQ-COOH) |
| L-IB1(s3) | 35 | 13 | PTTLNLFPQVPRS (NH₂-PTTLNLFPQVPRS-COOH) |
| L-IB1(s4) | 36 | 13 | RPTTLNLFPQVPR (NH₂-RPTTLNLFPQVPR-COOH) |
| L-IB1(s5) | 37 | 13 | KRPTTLNLFPQVP (NH₂-KRPTTLNLFPQVP-COOH) |
| L-IB1(s6) | 38 | 13 | PKRPTTLNLFPQV (NH₂-PKRPTTLNLFPQV-COOH) |
| L-IB1(s7) | 39 | 13 | RPKRPTTLNLFPQ (NH₂-RPKRPTTLNLFPQ-COOH) |
| L-IB1(s8) | 40 | 12 | LNLFPQVPRSQD (NH₂-LNLFPQVPRSQD-COOH) |
| L-IB1(s9) | 41 | 12 | TLNLFPQVPRSQ (NH₂-TLNLFPQVPRSQ-COOH) |
| L-IB1(s10) | 42 | 12 | TTLNLFPQVPRS (NH₂-TTLNLFPQVPRS-COOH) |
| L-IB1(s11) | 43 | 12 | PTTLNLFPQVPR (NH₂-PTTLNLFPQVPR-COOH) |
| L-IB1(s12) | 44 | 12 | RPTTLNLFPQVP (NH₂-RPTTLNLFPQVP-COOH) |
| L-IB1(s13) | 45 | 12 | KRPTTLNLFPQV (NH₂-KRPTTLNLFPQV-COOH) |
| L-IB1(s14) | 46 | 12 | PKRPTTLNLFPQ (NH₂-PKRPTTLNLFPQ-COOH) |
| L-IB1(s15) | 47 | 12 | RPKRPTTLNLFP (NH₂-RPKRPTTLNLFP-COOH) |
| L-IB1(s16) | 48 | 11 | NLFPQVPRSQD (NH₂-NLFPQVPRSQD-COOH) |
| L-IB1(s17) | 49 | 11 | LNLFPQVPRSQ (NH₂-LNLFPQVPRSQ-COOH) |
| L-IB1(s18) | 50 | 11 | TLNLFPQVPRS (NH₂-TLNLFPQVPRS-COOH) |
| L-IB1(s19) | 51 | 11 | TTLNLFPQVPR (NH₂-TTLNLFPQVPR-COOH) |
| L-IB1(s20) | 52 | 11 | PTTLNLFPQVP (NH₂-PTTLNLFPQVP-COOH) |
| L-IB1(s21) | 53 | 11 | RPTTLNLFPQV (NH₂-RPTTLNLFPQV-COOH) |
| L-IB1(s22) | 54 | 11 | KRPTTLNLFPQ (NH₂-KRPTTLNLFPQ-COOH) |
| L-IBi(s23) | 55 | 11 | PKRPTTLNLFP (NH₂-PKRPTTLNLFP-COOH) |
| L-1B1(s24) | 56 | 11 | RPKRPTTLNLF (NH₂-RPKRPTTLNLF-COOH) |
| L-IB1(s25) | 57 | 10 | LFPQVPRSQD (NH₂-LFPQVPRSQD-COOH) |
| L-IB1(s26) | 58 | 10 | NLFPQVPRSQ (NH₂-NLFPQVPRSQ-COOH) |
| L-IB1(s27) | 59 | 10 | LNLFPQVPRS (NH₂-LNLFPQVPRS-COOH) |
| L-IB1(s28) | 60 | 10 | TLNLFPQVPR (NH₂-TLNLFPQVPR-COOH) |
| L-IB1(s29) | 61 | 10 | TTLNLFPQVP (NH₂-TTLNLFPQVP-COOH) |
| L-IB1(s30) | 62 | 10 | PTTLNLFPQV (NH₂-PTTLNLFPQV-COOH) |
| L-IB1(s31) | 63 | 10 | RPTTLNLFPQ (NH₂-RPTTLNLFPQ-COOH) |
| L-IB1(s32) | 64 | 10 | KRPTTLNLFP (NH₂-KRPTTLNLFP-COOH) |
| L-IB1(s33) | 65 | 10 | PKRPTTLNLF (NH₂-PKRPTTLNLF-COOH) |
| L-IB1(s34) | 66 | 10 | RPKRPTTLNL (NH₂-RPKRPTTLNL-COOH) |
| D-IB1(s1) | 67 | 13 | QPFLNLTTPRKPR (NH₂-QPFLNLTTPRKPR-COOH) |
| D-IB1(s2) | 68 | 13 | VQPFLNLTTPRKP (NH₂-VQPFLNLTTPRKP-COOH) |
| D-IB1(s3) | 69 | 13 | PVQPFLNLTTPRK (NH₂-PVQPFLNLTTPRK-COOH) |
| D-IB1(s4) | 70 | 13 | RPVQPFLNLTTPR (NH₂-RPVQPFLNLTTPR-COOH) |
| D-IB1(s5) | 71 | 13 | SRPVQPFLNLTTP (NH₂-SRPVQPFLNLTTP-COOH) |
| D-IB1(s6) | 72 | 13 | QSRPVQPFLNLTT (NH₂-QSRPVQPFLNLTT-COOH) |
| D-IB1(s7) | 73 | 13 | DQSRPVQPFLNLT (NH₂-DQSRPVQPFLNLT-COOH) |
| D-IB1(s8) | 74 | 12 | PFLNLTTPRKPR (NH₂-PFLNLTTPRKPR-COOH) |
| D-IB1(s9) | 75 | 12 | QPFLNLTTPRKP (NH₂-QPFLNLTTPRKP-COOH) |
| D-IB1(s10) | 76 | 12 | VQPFLNLTTPRK (NH₂-VQPFLNLTTPRK-COOH) |
| D-IB1(s11) | 77 | 12 | PVQPFLNLTTPR (NH₂-PVQPFLNLTTPR-COOH) |
| D-IB1(s12) | 78 | 12 | RPVQPFLNLTTP (NH₂-RPVQPFLNLTTP-COOH) |
| D-IB1(s13) | 79 | 12 | SRPVQPFLNLTT (NH₂-SRPVQPFLNLTT-COOH) |
| D-IB1(s14) | 80 | 12 | QSRPVQPFLNLT (NH₂-QSRPVQPFLNLT-COOH) |
| D-IB1(s15) | 81 | 12 | DQSRPVQPFLNL (NH₂-DQSRPVQPFLNL-COOH) |
| D-IB1(s16) | 82 | 11 | FLNLTTPRKPR (NH₂-FLNLTTPRKPR-COOH) |
| D-IB1(s17) | 83 | 11 | PFLNLTTPRKP (NH₂-PFLNLTTPRKP-COOH) |
| D-IB1(s18) | 84 | 11 | QPFLNLTTPRK (NH₂-QPFLNLTTPRK-COOH) |
| D-IB1(s19) | 85 | 11 | VQPFLNLTTPR (NH₂-VQPFLNLTTPR-COOH) |
| D-IB1(s20) | 86 | 11 | PVQPFLNLTTP (NH₂-PVQPFLNLTTP-COOH) |
| D-IB1(s21) | 87 | 11 | RPVQPFLNLTT (NH₂-RPVQPFLNLTT-COOH) |
| D-IB1(s22) | 88 | 11 | SRPVQPFLNLT (NH₂-SRPVQPFLNLT-COOH) |
| D-IB1(s23) | 89 | 11 | QSRPVQPFLNL (NH₂-QSRPVQPFLNL-COOH) |
| D-IB1(s24) | 90 | 11 | DQSRPVQPFLN (NH₂-DQSRPVQPFLN-COOH) |
| D-IB1(s25) | 91 | 10 | DQSRPVQPFL (NH₂-DQSRPVQPFL-COOH) |
| D-IB1(s26) | 92 | 10 | QSRPVQPFLN (NH₂-QSRPVQPFLN-COOH) |
| D-IB1(s27) | 93 | 10 | SRPVQPFLNL (NH₂-SRPVQPFLNL-COOH) |
| D-IB1(s28) | 94 | 10 | RPVQPFLNLT (NH₂-RPVQPFLNLT-COOH) |
| D-IB1(s29) | 95 | 10 | PVQPFLNLTT (NH₂-PVQPFLNLTT-COOH) |
| D-IBi(s30) | 96 | 10 | VQPFLNLTTP (NH₂-VQPFLNLTTP-COOH) |
| D-IB1(s31) | 97 | 10 | QPFLNLTTPR (NH₂-QPFLNLTTPR-COOH) |
| D-IB1(s32) | 98 | 10 | PFLNLTTPRK (NH₂-PFLNLTTPRK-COOH) |
| D-IB1(s33) | 99 | 10 | FLNLTTPRKP (NH₂-FLNLTTPRKP-COOH) |
| D-IB1(s34) | 100 | 10 | LNLTTPRKPR (NH₂-LNLTTPRKPR-COOH) |

In the above context, an amino acid sequence having a sequence "sharing a sequence identity" of at least, for example, 95% to a query amino acid sequence of the present invention, is intended to mean that the sequence of the subject amino acid sequence is identical to the query sequence except that the subject amino acid sequence may include up to five amino acid alterations per each 100 amino acids of the query amino acid sequence. In other words, to obtain an amino acid sequence having a sequence of at least 95% identity to a query amino acid sequence, up to 5% (5 of 100) of the amino acid residues in the subject sequence may be inserted or substituted with another amino acid or deleted.

For sequences without exact correspondence, a "% identity" of a first sequence may be determined with respect to a second sequence. In general, these two sequences to be compared are aligned to give a maximum correlation between the sequences. This may include inserting "gaps" in either one or both sequences, to enhance the degree of alignment. A % identity may then be determined over the whole length of each of the sequences being compared (so-called global alignment), that is particularly suitable for sequences of the same or similar length, or over shorter, defined lengths (so-called local alignment), that is more suitable for sequences of unequal length.

Methods for comparing the identity and homology of two or more sequences, particularly as described herein, are well known in the art. Thus for instance, programs available in the Wisconsin Sequence Analysis Package, version 9.1 (Devereux et al., 1984, Nucleic Acids Res. 12, 387-395.), for example the programs BESTFIT and GAP, may be used to determine the % identity between two polynucleotides and the % identity and the % homology between two polypeptide sequences. BESTFIT uses the "local homology" algorithm of (Smith and Waterman (1981), J. Mol. Biol. 147, 195-197.) and finds the best single region of similarity between two sequences. Other programs for determining identity and/or similarity between sequences are also known in the art, for instance the BLAST family of programs (Altschul et al., 1990, J. Mol. Biol. 215, 403-410), accessible through the home page of the NCBI at world wide web site ncbi.nlm.nih.gov) and FASTA (Pearson (1990), Methods Enzymol. 183, 63-98; Pearson and Lipman (1988), Proc. Natl. Acad. Sci. U. S. A 85, 2444-2448.).

JNK-inhibitor sequences as used according to the present invention and as defined above may be obtained or produced by methods well-known in the art, e.g. by chemical synthesis or by genetic engineering methods as discussed below. For example, a peptide corresponding to a portion of an JNK inhibitor sequence as used herein including a desired region of said JNK inhibitor sequence, or that mediates the desired activity *in vitro* or *in vivo,* may be synthesized by use of a peptide synthesizer.

JNK inhibitor sequence as used herein and as defined above, may be furthermore be modified by a trafficking sequence, allowing the JNK inhibitor sequence as used herein and as defined above to be transported effectively into the cells. Such modified JNK inhibitor sequence are preferably provided and used as chimeric sequences.

The present invention therefore provides a chimeric peptide including at least one first domain and at least one second domain, for use in the treatment of diseases or disorders strongly related to JNK signaling as defined above in a subject, wherein the first domain of the chimeric peptide comprises a trafficking sequence, while the second domain of the chimeric peptide comprises an JNK inhibitor sequence according to SEQ ID NO: 2.

As a first domain the chimeric peptide as used herein comprises a trafficking sequence, which is typically selected from any sequence of amino acids that directs a peptide (in which it is present) to a desired cellular destination. Thus, the trafficking sequence, as used herein, typically directs the peptide across the plasma membrane, e.g. from outside the cell, through the plasma membrane, and into the cytoplasm. Alternatively, or in addition, the trafficking sequence may direct the peptide to a desired location within the cell, e.g. the nucleus, the ribosome, the endoplasmic reticulum (ER), a lysosome, or peroxisome, by e.g. combining two components (e.g. a component for cell permeability and a component for nuclear location) or by one single component having e.g. properties of cell membrane transport and targeted e.g. intranuclear transport. The trafficking sequence may additionally comprise another component, which is capable of binding a cytoplasmic component or any other component or compartment of the cell (e.g. endoplasmic reticulum, mitochondria, gloom apparatus, lysosomal vesicles). Accordingly, e.g. the trafficking sequence of the first domain and the JNK inhibitor sequence of the second domain may be localized in the cytoplasm or any other compartment of the cell. This allows to determine localization of the chimeric peptide in the cell upon uptake.

The trafficking sequence of the chimeric peptide as used herein may be exclusively composed of D-amino acids. More preferably, the trafficking sequence of the chimeric peptide as used herein may comprise a D retro-inverso peptide of the sequences as presented above.

The trafficking sequence of the first domain of the chimeric peptide as described herein may be obtained from naturally occurring sources or can be produced by using genetic engineering techniques or chemical synthesis (see e.g. Sambrook, J., Fritsch, E. F., Maniatis, T. (1989) Molecular cloning: A laboratory manual. 2nd edition. Cold Spring Harbor Laboratory Press, Cold Spring Harbor, N.Y.).

Sources for the trafficking sequence of the first domain may be employed including, e.g. native proteins such as e.g. the TAT protein (e.g. as described in U.S. Patent Nos. 5,804,604 and 5,674,980, each of these references being incorporated herein by reference), VP22 (described in e.g. WO 97/05265; Elliott and O'Hare, Cell 88 : 223-233 (1997)), non-viral proteins (Jackson et al, Proc. Natl. Acad. Sci. USA 89 : 10691-10695 (1992)), trafficking sequences derived from Antennapedia (e.g. the antennapedia carrier sequence) or from basic peptides, e.g. peptides having a length of 5 to 15 amino acids, preferably 10 to 12 amino acids and comprising at least 80 %, more preferably 85 % or even 90 % basic amino acids, such as e.g. arginine, lysine and/or histidine. Furthermore, variants, fragments and derivatives of one of the native proteins used as trafficking sequences are disclosed herewith. With regard to variants, fragments and derivatives it is referred to the definition given above for JNK inhibitor sequences as described herein. Variants, fragments as well as derivatives are correspondingly defined as set forth above for JNK inhibitor sequences as described herein. Particularly, in the context of the trafficking sequence, a variant or fragment or derivative may be defined as a sequence sharing a sequence identity with one of the native proteins used as trafficking sequences as defined above of at least about 30%, 50%, 70%, 80%, 90%, 95%, 98%, or even 99%.

In a chimeric peptide as described herein, the trafficking sequence of the first domain comprises or consists of a sequence derived from the human immunodeficiency virus (HIV)1 TAT protein, particularly some or all of the 86 amino acids that make up the TAT protein.

For a trafficking sequence (being included in the first domain of the chimeric peptide as used herein), partial sequences of the full-length TAT protein may be used forming a functionally effective fragment of a TAT protein, i.e. a TAT peptide that includes the region that mediates entry and uptake into cells. As to whether such a sequence is a functionally effective fragment of the TAT protein can be determined using known techniques (see e.g. Franked et al., Proc. Natl. Acad. Sci, USA 86 : 7397-7401 (1989)). Thus, the trafficking sequence in the first domain of the chimeric peptide as used herein may be derived from a functionally effective fragment or portion of a TAT protein sequence that comprises less than 86 amino acids, and which exhibits uptake into cells, and optionally the uptake into the cell nucleus. More preferably, partial sequences (fragments) of TAT to be used as carrier to mediate permeation of the chimeric peptide across the cell membrane, are intended to comprise the basic region (amino acids 48 to 57 or 49 to 57) of full-length TAT.

The trafficking sequence as used herein comprises the D retro-inverso sequence NH₂-RRRQRRKKRG-COOH (D-TAT) [SEQ ID NO: 6].

As a second domain the chimeric peptide as described herein typically comprises an JNK inhibitor sequence, selected from any of the JNK inhibitor sequences as defined above, including variants, fragments and/or derivatives of these JNK inhibitor sequences.

Both domains, i.e. the first and the second domain(s), of the chimeric peptide as used herein, may be linked such as to form a functional unit. Any method for linking the first and second domain(s) as generally known in the art may be applied.

The first and the second domain(s) of the chimeric peptide as used herein are linked by a covalent bond. A covalent bond, as defined herein, may be e.g. a peptide bond, which may be obtained by expressing the chimeric peptide as defined above as a fusion protein. Fusion proteins, as described herein, can be formed and used in ways analogous to or readily adaptable from standard recombinant DNA techniques, as described below. However, both domains may also be linked via side chains or may be linked by a chemical linker moiety.

The first and second domain(s) may be linked with each other via a linker sequence comprising 1 to 10, preferably 1 to 5 amino acids. Amino acids forming the linker sequence are preferably selected from glycine or proline as amino acid residues. More preferably, the first and second domain(s) may be separated by each other by a hinge of two, three or more proline residues between the first and second domain(s).

The chimeric peptide as used herein consists of D-amino acid chimeric peptides according to the TAT-IB1 peptide NH₂-DQSRPVQPFLNLTTPRKPRPPRRRQRRKKRG-COOH (D-TAT-IB1(s)) [SEQ ID NO: 11].

The first and second domain(s) of the chimeric peptide as defined above may be linked to each other by chemical or biochemical coupling carried out in any suitable manner known in the art, e.g. by establishing a peptide bond between the first and the second domain(s) e.g. by expressing the first and second domain(s) as a fusion protein, or e.g. by crosslinking the first and second domain(s) of the chimeric peptide as defined above.

Many known methods suitable for chemical crosslinking of the first and second domain(s) of the chimeric peptide as defined above are non-specific, i.e. they do not direct the point of coupling to any particular site on the transport polypeptide or cargo macromolecule. As a result, use of non-specific crosslinking agents may attack functional sites or sterically block active sites, rendering the conjugated proteins biologically inactive. Thus, preferably such crosslinking methods are used, which allow a more specific coupling of the first and second domain(s).

In this context, one way to increasing coupling specificity is a direct chemical coupling to a functional group present only once or a few times in one or both of the first and second domain(s) to be crosslinked. For example, cysteine, which is the only protein amino acid containing a thiol group, occurs in many proteins only a few times. Also, for example, if a polypeptide contains no lysine residues, a crosslinking reagent specific for primary amines will be selective for the amino terminus of that polypeptide. Successful utilization of this approach to increase coupling specificity requires that the polypeptide have the suitably rare and reactive residues in areas of the molecule that may be altered without loss of the molecule's biological activity. Cysteine residues may be replaced when they occur in parts of a polypeptide sequence where their participation in a crosslinking reaction would otherwise likely interfere with biological activity.

When a cysteine residue is replaced, it is typically desirable to minimize resulting changes in polypeptide folding. Changes in polypeptide folding are minimized when the replacement is chemically and sterically similar to cysteine. For these reasons, serine is preferred as a replacement for cysteine. As demonstrated in the examples below, a cysteine residue may be introduced into a polypeptide's amino acid sequence for crosslinking purposes. When a cysteine residue is introduced, introduction at or near the amino or carboxy terminus is preferred. Conventional methods are available for such amino acid sequence modifications, wherein the polypeptide of interest is produced by chemical synthesis or via expression of recombinant DNA.

Coupling of the first and second domain(s) of the chimeric peptide as defined above and used herein can also be accomplished via a coupling or conjugating agent. There are several intermolecular crosslinking reagents which can be utilized (see for example, Means and Feeney, CHEMICAL MODIFICATION OF PROTEINS, Holden-Day, 1974, pp. 39-43). Among these reagents are, for example, N-succinimidyl 3-(2-pyridyldithio) propionate (SPDP) or N,N'-(1,3-phenylene) bismaleimide (both of which are highly specific for sulfhydryl groups and form irreversible linkages); N, N'-ethylene-bis-(iodoacetamide) or other such reagent having 6 to 11 carbon methylene bridges (which are relatively specific for sulfhydryl groups); and 1,5-difluoro-2,4-dinitrobenzene (which forms irreversible linkages with amino and tyrosine groups). Other crosslinking reagents useful for this purpose include: p,p'-difluoro-m, m'-dinitrodiphenylsulfone which forms irreversible crosslinkages with amino and phenolic groups); dimethyl adipimidate (which is specific for amino groups); phenol-1,4 disulfonylchloride (which reacts principally with amino groups); hexamethylenediisocyanate or diisothiocyanate, or azophenyl-p-diisocyanate (which reacts principally with amino groups); glutaraldehyde (which reacts with several different side chains) and disdiazobenzidine (which reacts primarily with tyrosine and histidine).

Crosslinking reagents used for crosslinking the first and second domain(s) of the chimeric peptide as defined above may be homobifunctional, i.e. having two functional groups that undergo the same reaction. A preferred homobifunctional crosslinking reagent is bismaleimidohexane ("BMH"). BMH contains two maleimide functional groups, which react specifically with sulfhydryl-containing compounds under mild conditions (pH 6.5-7.7). The two maleimide groups are connected by a hydrocarbon chain. Therefore, BMH is useful for irreversible crosslinking of polypeptides that contain cysteine residues.

Crosslinking reagents used for crosslinking the first and second domain(s) of the chimeric peptide as defined above may also be heterobifunctional. Heterobifunctional crosslinking agents have two different functional groups, for example an amine-reactive group and a thiol-reactive group, that will crosslink two proteins having free amines and thiols, respectively. Examples of heterobifunctional crosslinking agents are succinimidyl 4-(N-maleimidomethyl)cyclohexane-1-carboxylate ("SMCC"), m-maleimidobenzoyl-N-hydroxysuccinimide ester ("MBS"), and succinimide 4-(p-maleimidophenyl)butyrate ("SMPB"), an extended chain analog of MBS. The succinimidyl group of these crosslinkers reacts with a primary amine, and the thiol-reactive maleimide forms a covalent bond with the thiol of a cysteine residue.

Crosslinking reagents suitable for crosslinking the first and second domain(s) of the chimeric peptide as defined above often have low solubility in water. A hydrophilic moiety, such as a sulfonate group, may thus be added to the crosslinking reagent to improve its water solubility. In this respect, Sulfo-MBS and Sulfo-SMCC are examples of crosslinking reagents modified for water solubility, which may be used according to the present invention.

Likewise, many crosslinking reagents yield a conjugate that is essentially non-cleavable under cellular conditions. However, some crosslinking reagents particularly suitable for crosslinking the first and second domain(s) of the chimeric peptide as defined above contain a covalent bond, such as a disulfide, that is cleavable under cellular conditions. For example, Traut's reagent, dithiobis(succinimidylpropionate) ("DSP"), and N-succinimidyl 3-(2-pyridyldithio)propionate ("SPDP") are well-known cleavable crosslinkers. The use of a cleavable crosslinking reagent permits the cargo moiety to separate from the transport polypeptide after delivery into the target cell. Direct disulfide linkage may also be useful.

Numerous crosslinking reagents, including the ones discussed above, are commercially available. Detailed instructions for their use are readily available from the commercial suppliers. A general reference on protein crosslinking and conjugate preparation is: Wong, CHEMISTRY OF PROTEIN CONJUGATION AND CROSSLINKING, CRC Press (1991).

Chemical crosslinking of the first and second domain(s) of the chimeric peptide as defined above may include the use of spacer arms. Spacer arms provide intramolecular flexibility or adjust intramolecular distances between conjugated moieties and thereby may help preserve biological activity. A spacer arm may be in the form of a polypeptide moiety that includes spacer amino acids, e.g. proline. Alternatively, a spacer arm may be part of the crosslinking reagent, such as in "long-chain SPDP" (Pierce Chem. Co., Rockford, IL., cat. No. 21651 H).

Any of the peptides disclosed herein, in particular the JNK inhibitor, the trafficking sequence and the chimeric peptide as disclosed herein, preferably the JNK inhibitor according to SEQ ID NO: 11, may have a modification at one or both of their termini, i.e. either at the C- or at the N-terminus or at both. The C-Terminus may preferably be modified by an amide modification, whereas the N-terminus may be modified by any suitable NH2-protection group, such as e.g. acylation. More preferably, the JNK inhibitor and the chimeric peptide as disclosed herein, preferably the JNK inhibitor according to SEQ ID NO: 11, is modified by an amide modification at the C-terminus.

Any of the peptides disclosed herein, in particular the JNK inhibitor, the trafficking sequence (e.g. of the chimeric peptide) and the chimeric peptide as disclosed herein, preferably the JNK inhibitor according to SEQ ID NO: 11, may be deleted at their N- and/or C-terminus by 1, 2 or 3 amino acids. For example, in a chimeric peptide each domain, i.e. the JNK-inhibitor and the trafficking sequence domain, may be deleted at their N- and/or C-terminus by 1, 2 or 3 amino acids and/or the chimeric peptide may be deleted at its N- and/or C-terminus by 1, 2 or 3 amino acids. Again, the shorter the peptides are, the less their (unspecific) cell toxicity. However, the peptides must retain their biological function, i.e. their cell membrane permeability (first domain) and their JNK inhibitory function (second domain).

Furthermore, variants, fragments or derivatives of one of the above disclosed chimeric peptides are described herein. With regard to fragments and variants it is generally referred to the definition given above for JNK inhibitor sequences.

Particularly, a "variant of a chimeric peptide" is preferably a sequence derived from any of the sequences according to SEQ ID NOs: 9 to 12 and 23 to 32, wherein the chimeric variant comprises amino acid alterations of the chimeric peptides according to SEQ ID NOs: 9 to 12 and 23 to 32 as used herein. Such alterations typically comprise 1 to 20, preferably 1 to 10 and more preferably 1 to 5 substitutions, additions and/or deletions (leading to fragments) of amino acids according to SEQ ID NOs: 9 to 12 and 23 to 32, wherein the altered chimeric peptide as described herein exhibits a sequence identity with any of the sequences according to SEQ ID NOs: 9-12 and 23 to 32 of at least about 30%, 50%, 70%, 80%, or 95%, 98%, or even 99%. Preferably, these variants retain the biological activity of the first and the second domain as contained in the chimeric peptide, i.e. the trafficking activity of the first domain as disclosed above and the activity of the second domain for binding JNK and/or inhibiting the activation of at least one JNK activated transcription factor.

Thus, in the context of the present invention, a "fragment of the chimeric peptide" is preferably a sequence derived any of the sequences according to SEQ ID NOs: 9 to 12 and 23 to 32, wherein the fragment comprises at least 4 contiguous amino acids of any of SEQ ID NOs: 9 to 12 and 23 to 32. This fragment preferably comprises a length which is sufficient to allow specific recognition of an epitope from any of these sequences and to transport the sequence into the cells, the nucleus or a further preferred location. Even more preferably, the fragment comprises 4 to 18, 4 to 15, or most preferably 4 to 10 contiguous amino acids of any of SEQ ID NOs: 9 to 12 and 23 to 32. Fragments of the chimeric peptide as described herein further may be defined as a sequence sharing a sequence identity with any of the sequences according to any of SEQ ID NOs: 9 to 12 and 23 to 32 of at least about 30%, 50%, 70%, 80%, or 95%, 98%, or even 99%.

The chimeric peptides as defined according to the invention can be formulated in a pharmaceutical composition, which may be applied in the prevention or treatment of any of the diseases as defined herein, particularly in the prevention or treatment of diseases or disorders strongly related to JNK signaling as defined herein. Such a pharmaceutical composition used according to the present invention includes as an active component (i) chimeric peptides according to SEQ ID NO: 11 according to the invention.

According to a preferred embodiment, such a pharmaceutical composition according to the present invention typically comprises a safe and effective amount of a component according to the invention, of at least one chimeric peptide according to SEQ ID NO: 11. A pharmaceutical composition according to the present invention comprises as an active component a chimeric peptide consisting of the sequence according to SEQ ID NO: 11.

In addition, the pharmaceutical composition as used according to the present invention may additionally - i.e. in addition to any one or more of the chimeric peptides according to the invention- also comprise optionally a further "active component", which is also useful in the respective disease. In this context, the pharmaceutical composition according to the present invention may also combined in the therapy of the diseases according to the present invention with a further pharmaceutical composition comprising a further "active component". In the case of a combination therapy, separate pharmaceutical compositions for the active components to be combined are preferred for better individual dosing, however for convenience also a single pharmaceutical composition comprising the active components to be combined is conceivable. In the case of separate pharmaceutical compositions for the active components to be combined the administration of the JNK inhibitor according to the present invention may be before, during (concomitant or overlapping administration) or after the administration of the other active component comprised in a separate pharmaceutical composition. Administration "before" the administration of the JNK inhibitor preferably means within 24 h, more preferably within 12 h, even more preferably within 3 h, particularly preferably within 1 h and most preferably within 30 min before the administration of the JNK inhibitor starts. Administration "after" the administration of the JNK inhibitor preferably means within 24 h, more preferably within 12 h, even more preferably within 3 h, particularly preferably within 1 h and most preferably within 30 min after the administration of the JNK inhibitor is finished.

The inventors of the present invention additionally found, that the the chimeric peptide, as defined herein, exhibit a particular well uptake rate into cells involved in the diseases of the present invention. Therefore, the amount of a chimeric peptide, in the pharmaceutical composition to be administered to a subject, may -without being limited thereto - have a very low dose. Thus, the dose may be much lower than for peptide drugs known in the art, such as DTS-108 (Florence Meyer-Losic et al., Clin Cancer Res., 2008, 2145-53). This has several positive aspects, for example a reduction of potential side reactions and a reduction in costs.

Preferably, the dose (per kg bodyweight) is in the range of up to 10 mmol/kg, preferably up to 1 mmol/kg, more preferably up to 100 µmοl/kg, even more preferably up to 10 µmοl/kg, even more preferably up to 1 µmοl/kg, even more preferably up to 100 nmol/kg, most preferably up to 50 nmol/kg.

Thus, the dose range may preferably be from about 0,01 pmol/kg to about 1 mmol/kg, from about 0,1 pmol/kg to about 0,1 mmol/kg, from about 1,0 pmol/kg to about 0,01 mmol/kg, from about 10 pmol/kg to about 1 µmol/kg, from about 50 pmol/kg to about 500 nmol/kg, from about 100 pmol/kg to about 300 nmol/kg, from about 200 pmol/kg to about 100 nmol/kg, from about 300 pmol/kg to about 50 nmol/kg, from about 500 pmol/kg to about 30 nmol/kg, from about 250 pmol/kg to about 5 nmol/kg, from about 750 pmol/kg to about 10 nmol/kg, from about 1 nmol/kg to about 50 nmol/kg, or a combination of any two of said values.

In this context, prescription of treatment, e.g. decisions on dosage etc. when using the above pharmaceutical composition is typically within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in REMINGTON'S PHARMACEUTICAL SCIENCES, 16th edition, Osol, A. (ed), 1980. Accordingly, a "safe and effective amount" as defined above for components of the pharmaceutical compositions as used according to the present invention means an amount of each or all of these components, that is sufficient to significantly induce a positive modification of diseases or disorders strongly related to JNK signaling as defined herein. At the same time, however, a "safe and effective amount" is small enough to avoid serious side-effects, that is to say to permit a sensible relationship between advantage and risk. The determination of these limits typically lies within the scope of sensible medical judgment. A "safe and effective amount" of such a component will vary in connection with the particular condition to be treated and also with the age and physical condition of the patient to be treated, the severity of the condition, the duration of the treatment, the nature of the accompanying therapy, of the particular pharmaceutically acceptable carrier used, and similar factors, within the knowledge and experience of the accompanying doctor. The pharmaceutical compositions according to the invention can be used according to the invention for human and also for veterinary medical purposes.

The pharmaceutical composition according to the present invention may furthermore comprise, in addition to one of these substances, a (compatible) pharmaceutically acceptable carrier, excipient, buffer, stabilizer or other materials well known to those skilled in the art.

In this context, the expression "(compatible) pharmaceutically acceptable carrier" preferably includes the liquid or non-liquid basis of the composition. The term "compatible" means that the constituents of the pharmaceutical composition as used herein are capable of being mixed with the pharmaceutically active component as defined above and with one another component in such a manner that no interaction occurs which would substantially reduce the pharmaceutical effectiveness of the composition under usual use conditions. Pharmaceutically acceptable carriers must, of course, have sufficiently high purity and sufficiently low toxicity to make them suitable for administration to a person to be treated.

If the pharmaceutical composition as used herein is provided in liquid form, the pharmaceutically acceptable carrier will typically comprise one or more (compatible) pharmaceutically acceptable liquid carriers. The composition may comprise as (compatible) pharmaceutically acceptable liquid carriers e.g. pyrogen-free water; isotonic saline, i.e. a solution of 0.9 % NaCl, or buffered (aqueous) solutions, e.g. phosphate, citrate etc. buffered solutions, vegetable oils, such as, for example, groundnut oil, cottonseed oil, sesame oil, olive oil, corn oil and oil from theobroma; polyols, such as, for example, polypropylene glycol, glycerol, sorbitol, mannitol and polyethylene glycol; alginic acid, etc.. Particularly for injection and/or infusion of the pharmaceutical composition as used herein, a buffer, preferably an aqueous buffer, and/or 0.9 % NaCl may be used.

If the pharmaceutical composition as used herein is provided in solid form, the pharmaceutically acceptable carrier will typically comprise one or more (compatible) pharmaceutically acceptable solid carriers. The composition may comprise as (compatible) pharmaceutically acceptable solid carriers e.g. one or more compatible solid or liquid fillers or diluents or encapsulating compounds may be used as well, which are suitable for administration to a person. Some examples of such (compatible) pharmaceutically acceptable solid carriers are e.g. sugars, such as, for example, lactose, glucose and sucrose; starches, such as, for example, corn starch or potato starch; cellulose and its derivatives, such as, for example, sodium carboxymethylcellulose, ethylcellulose, cellulose acetate; powdered tragacanth; malt; gelatin; tallow; solid glidants, such as, for example, stearic acid, magnesium stearate; calcium sulphate, etc..

The precise nature of the (compatible) pharmaceutically acceptable carrier or other material may depend on the route of administration. The choice of a (compatible) pharmaceutically acceptable carrier may thus be determined in principle by the manner in which the pharmaceutical composition as used according to the invention is administered. Various possible routes of administration are listed in the list "Route of Administration" of the FDA (cf. FDA: Data Standards Manual - Drug Nomenclature Monographs - Monograph Number: C-DRG-00301; Version Number 004), which is incorporated by reference herein. Further guidance for selecting an appropriate route of administration, in particular for non-human animals, can be found in Turner PV et al. (2011) Journal of the American Association for Laboratory Animal Science, Vol. 50, No 5, p. 600 - 613, which is also incorporated by reference herein. Preferred examples for routes for administration include parenteral routes (e.g. via injection), such as intravenous, intramuscular, subcutaneous, intradermal, or transdermal routes, etc., enteral routes, such as oral, or rectal routes, etc., topical routes, such as nasal, or intranasal routes, etc., or other routes, such as epidermal routes or patch delivery. Also contemplated (in particular for eye related diseases) are instillation, intravitreal, and subconjunctival administration. Likewise, administration may occur intratympanical, for example, whenever ear related diseases are treated.

The route of administration of the JNK inhibitor according to the present invention, which is typically chosen according to the disease to be prevented and/or treated and the respective pharmacokinetics, is intravesical administration (i.e. into the urinary bladder), for example for diseases of the urinary system, in particular the urinary bladder.

In general, the method of administration depends on various factors as mentioned above, for example the selected pharmaceutical carrier and the nature of the pharmaceutical preparation (e.g. as a liquid, tablet etc.) as well as the route of administration. For example, the pharmaceutical composition comprising the JNK inhibitor according to the invention may be prepared as a liquid, for example as a solution of the chimeric peptide according to a sequence of SEQ ID NO. 11, in 0.9 % NaCl. Accordingly, for the administration different devices may be used, e.g. a syringe (including a pre-filled syringe); an injection device (e.g. the INJECT-EASET^{™} and GENJECTT^{™} device); an infusion pump (such as e.g. Accu-Chek^{™}); an injector pen (such as the GENPENT^{™}); a needleless device (e.g. MEDDECTOR^{™} and BIOJECTOR^{™}); or an autoinjector.

The suitable amount of the pharmaceutical composition to be used can be determined by routine experiments with animal models. Such models include, without implying any limitation, for example rabbit, sheep, mouse, rat, gerbil, dog, pig and non-human primate models. Preferred unit dose forms for administration include sterile solutions of water, physiological saline or mixtures thereof. The pH of such solutions should be adjusted to about 7.4. Suitable carriers for administration include hydrogels, devices for controlled or delayed release, polylactic acid and collagen matrices.

For injection and/or infusion at the site of affliction, i.e. local injection/infusion, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, in particular 0.9 % NaCl, Ringer's Injection, Lactated Ringer's Injection. Preservatives, stabilizers, buffers, antioxidants and/or other additives may be included, as required. Administration is preferably in a "prophylactically effective amount or a "therapeutically effective amount" (as the case may be), this being sufficient to show benefit to the individual. The actual amount administered, and rate and time-course of administration, will depend on the nature and severity of what is being treated.

The invention concern the chimeric peptide having a sequence according to SEQ ID NO. 11, in particular in a pharmaceutical composition as defined herein, for use in the prevention and/or treatment of the following diseases/disorders:
(i) diseases and/or disorders of the urinary system, i.e. cystitis in general, in particular interstitial cystitis, Hunner's ulcer, trigonitis and/or hemorrhagic cystitis; wherein the JNK inhibitor is applied intravesically, more preferably by intravesical infusion, preferably at a concentration of 10 µg/ml - 1000 mg/ml, more prefarbly 50 µg/ml - 500 mg/ml, even more preferably 100 µg/ml - 100 mg/ml, and particularly preferably 0.5 mg/ml - 50 mg/ml, preferably in single doses of 0.1 - 1000 mg, more preferably 0.5 - 500 mg, even more preferably 1 - 100 mg, and particularly preferably 2 - 10 mg, preferably administered in one single dose, however, if applicable also preferably administered repeatedly, for example daily, every 2 or 3 days or weekly, for several, e.g. 2, 3, 4, 5, 6, 7, 8, 9, or 10, weeks.

Prevention and/or treatment of cystitis as defined herein typically includes administration of a pharmaceutical composition as defined above. The term "modulate" includes the suppression of expression of JNK when it is over-expressed in the above disease. It also includes suppression of phosphorylation of c-jun, ATF2 or NFAT4 in the above disease by at least one chimeric peptide according to SEQ ID NO: 11 as a competitive inhibitor of the natural c-jun, ATF2 and NFAT4 binding site in a cell. The term "modulate" also includes suppression of hetero- and homomeric complexes of transcription factors made up of, without being limited thereto, c-jun, ATF2, or NFAT4 and their related partners, such as for example the AP-1 complex that is made up of c-jun, AFT2 and c-fos. When a disease or disorder strongly related to JNK signaling as defined above is associated with JNK overexpression, such suppressive JNK inhibitor sequences can be introduced to a cell. In some instances, "modulate" may then include the increase of JNK expression, for example by an IB peptide-specific antibody that blocks the binding of an IB-peptide to JNK, thus preventing JNK inhibition by the IB-related peptide.

Prevention and/or treatment of a subject with the pharmaceutical composition as disclosed above may be typically accomplished by administering *(in vivo)* an ("therapeutically effective") amount of said pharmaceutical composition to a subject, wherein the subject may be e.g. any mammal, e.g. a human, a primate, mouse, rat, dog, cat, cow, horse or pig, whereby a human is particularly preferred. The term "therapeutically effective" means that the active component of the pharmaceutical composition is of sufficient quantity to ameliorate the disease or disorder strongly related to JNK signaling as defined above.

Accordingly, the invention refers to the chimeric peptide according to SEQ ID NO: 11, for use in the treatment of diseases or disorders strongly related to JNK signaling as defined above, e.g. by modulating activated JNK signaling pathways.

The chimeric peptides according to SEQ ID NO: 11, may be utilized in (*in vitro*) assays (e.g. immunoassays) to detect, prognose, diagnose, or monitor various conditions and disease states selected from diseases or disorders strongly related to JNK signaling as defined above, or monitor the treatment thereof. The immunoassays that may be utilized include, but are not limited to, competitive and non-competitive assay systems using techniques such as Western Blots, radioimmunoassays (RIA), enzyme linked immunosorbent assay (ELISA), "sandwich" immunoassays, immunoprecipitation assays, precipitin reactions, gel diffusion precipitin reactions, immunodiffusion assays, agglutination assays, fluorescent immunoassays, complement-fixation assays, immunoradiometric assays, and protein-A immunoassays, etc.. Alternatively, (*in vitro)* assays may be performed by delivering chimeric peptides, as defined above, to target cells typically selected from e.g. cultured animal cells, human cells or micro-organisms, and to monitor the cell response by biophysical methods typically known to a skilled person. The target cells typically used therein may be cultured cells *(in vitro)* or *in vivo* cells, i.e. cells composing the organs or tissues of living animals or humans, or microorganisms found in living animals or humans.

Additionally, the use of kits for diagnostic or therapeutic purposes, particular for the treatment, prevention or monitoring of diseases or disorders strongly related to JNK signaling as defined above is described, wherein the kit includes one or more containers containing JNK inhibitor sequences. The kit may, optionally, further comprise a predetermined amount of a purified JNK inhibitor sequence as defined above, a chimeric peptide as defined above, or nucleic acids encoding these, for use as a diagnostic, standard, or control in the assays for the above purposes.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In the case of conflict, the present specification, including definitions, will control. In addition, the materials, methods, and examples are illustrative only and not intended to be limiting. Other features and advantages of the invention will be apparent from the following detailed description and claims.

### DESCRIPTION OF FIGURES

- Figure 1: are diagrams showing alignments of conserved JBD domain regions in the indicated transcription factors. JNK inhibitor sequences used herein were identified by carrying out sequence alignments. The results of this alignment are exemplarily shown in Figures 1A-1C. Figure 1A depicts the region of highest homology between the JBDs of IB1, IB2, c-Jun and ATF2. Panel B depicts the amino acid sequence of the JBDs of L-IB1(s) and L-IB1 for comparative reasons. Fully conserved residues are indicated by asterisks, while residues changed to Ala in the GFP-JBD_{23Mut} vector are indicated by open circles. Figure 1C shows the amino acid sequences of chimeric proteins that include a JNK inhibitor sequence and a trafficking sequence. In the example shown, the trafficking sequence is derived from the human immunodeficiency virus (HIV) TAT polypeptide, and the JNK inhibitor sequence is derived from an IB1(s) polypeptide. Human, mouse, and rat sequences are identical in Panels B and C.

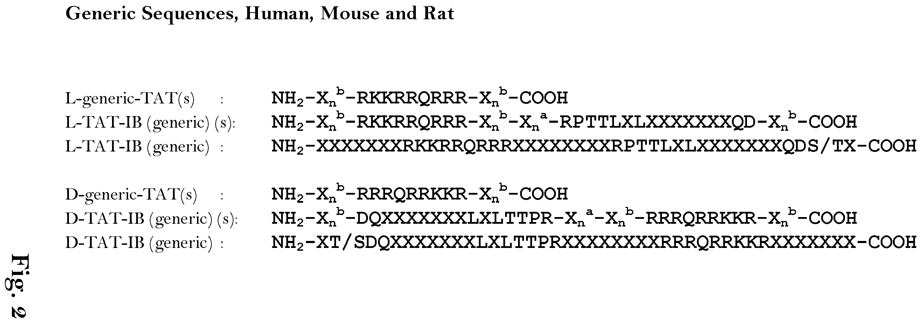

- Figure 2: is a diagram showing sequences of generic TAT-IB fusion peptides from human, mouse and rat.

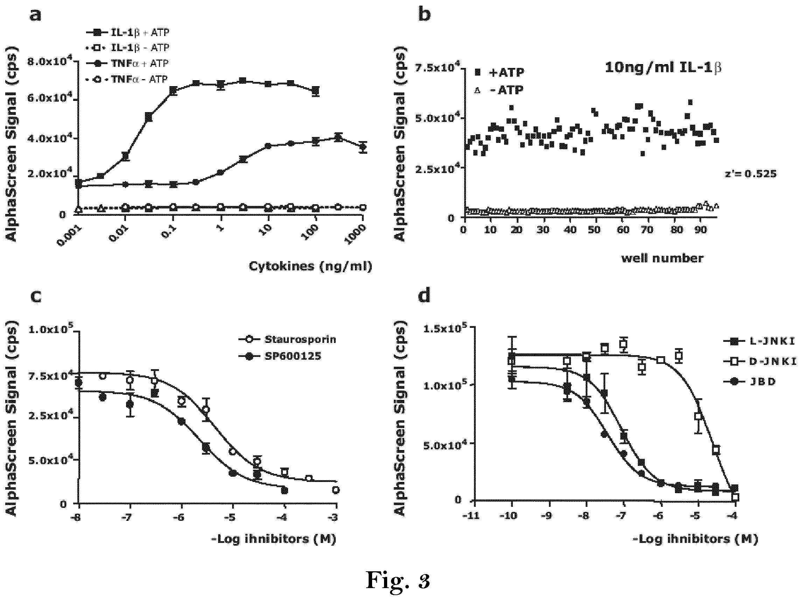

- Figure 3: depicts the results of the inhibition of endogeneous JNK-activity in HepG2 cells using fusion peptides according to SEQ ID NOs: 9 and 11 in an one-well approach. As can be seen from Figure 3, particularly panel d in Figure 3, D-TAT-IB1(s) according to SEQ ID NO: 11 (here abbreviated as D-JNKI) effectively inhibits JNK activity, even better than L-TAT-IB1(s) according to SEQ ID NO: 9 (here abbreviated as L-JNKI).

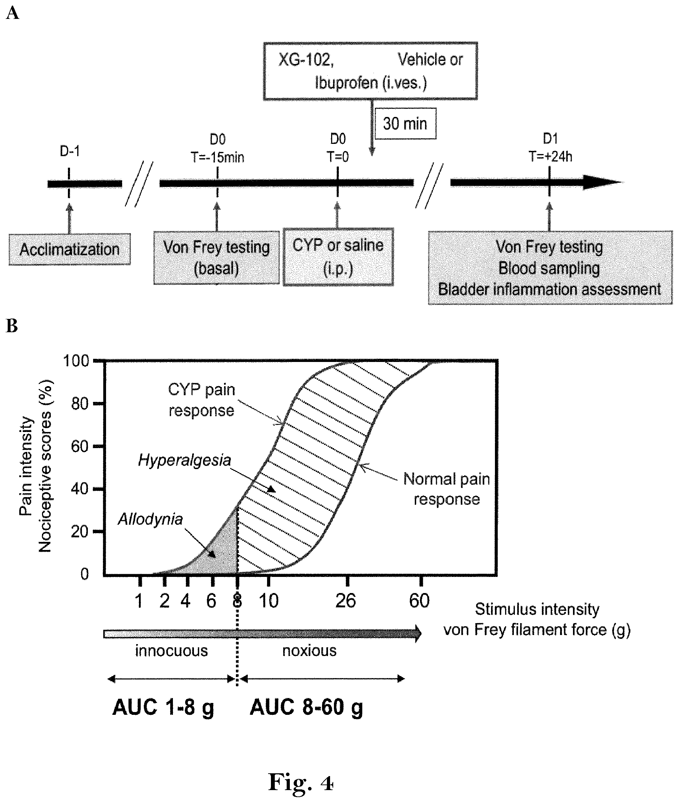

- Figure 4: shows for Example 11 the study design (A) and the AUCs method to assess allodynia and hyperalgesia (B).

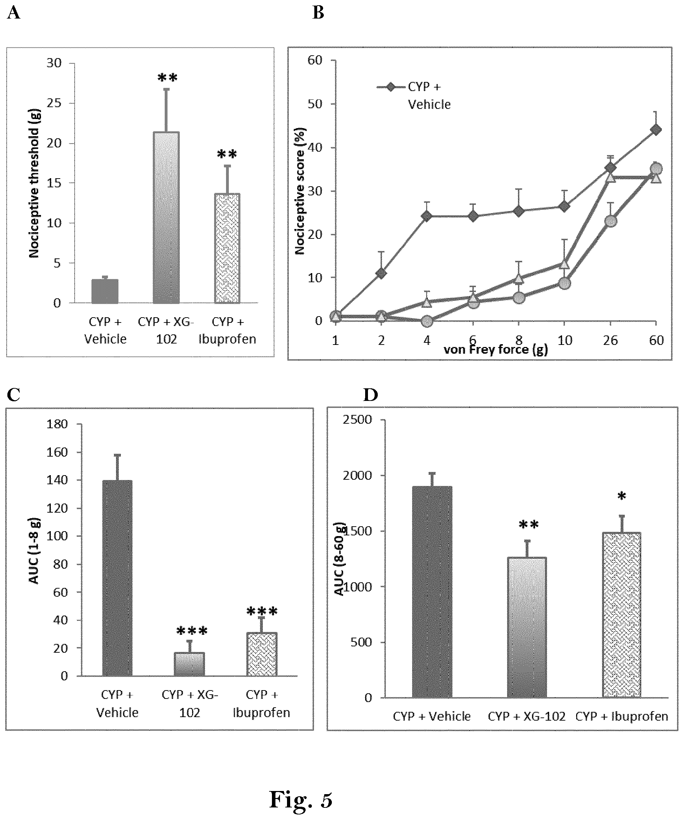

- Figure 5: shows for Example 11 the effect of XG-102 (50 mg/mL, i.ves.) and ibuprofen (50 mg/mL, i.ves.) treatments on nociceptive parameters 24h post-CYP injection. Nociceptive threshold (A), nociceptive scores (B), AUC 1-8 g (C) or AUC 8-60 g (D) 24h after CYP injection. Results are expressed as mean ± s.e.m. (n=10). 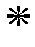 p<0.05, 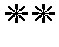 p<0.01, 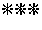 p<0.001 *vs* Vehicletreated group, Mann Whitney test (A and C), Two-way RM ANOVA (B), and Unpaired t test and Mann Whitney test (D).

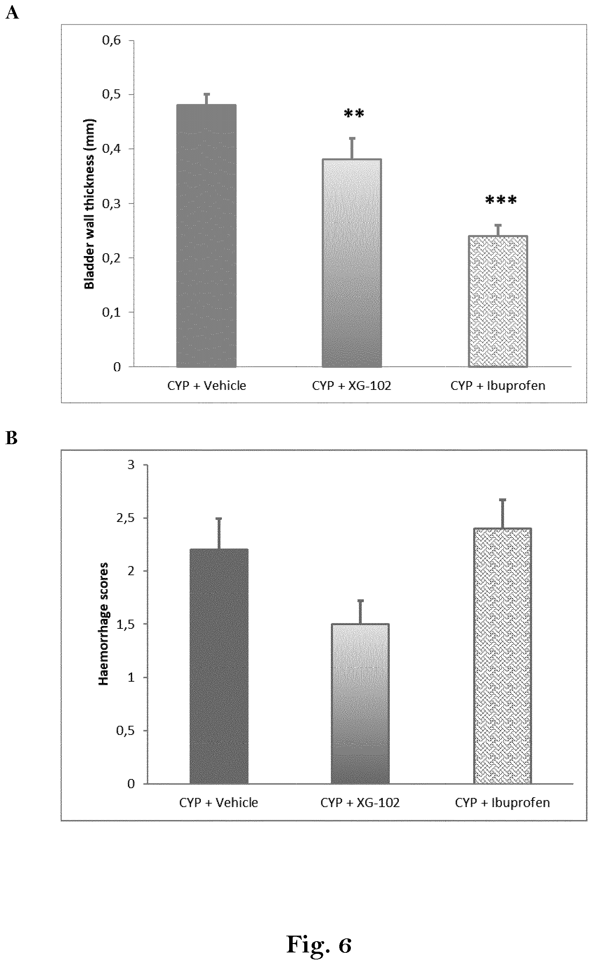

- Figure 6: shows for Example 11 the effect of XG-102 (50 mg/mL, i.ves.) and ibuprofen (50 mg/mL, i.ves.) treatments on urinary bladder wall thickness as well as haemorrhage scores 24h post-CYP injection. Urinary bladder wall thickness (A) or haemorrhage scores (B) 24h after CYP injection. Results are expressed as mean ± s.e.m. (n=10). ns= p>0.05, 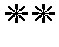 p<0.01, 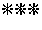 p<0.001 *vs* Vehicle-treated group, Mann Whitney test and Unpaired *t* test (A) or Mann Whitney test (B).

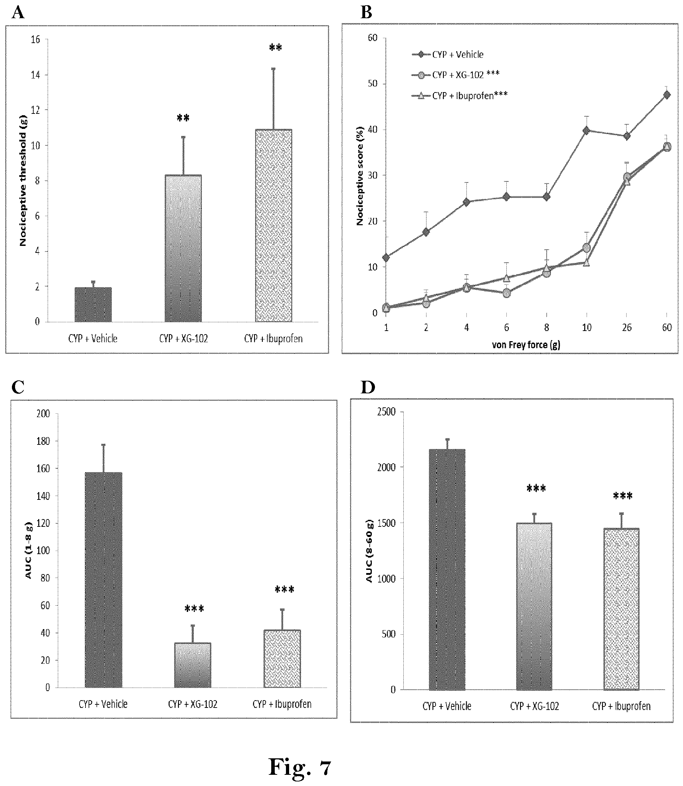

- Figure 7: shows for Example 12 the effect of XG-102 (2 mg/kg, i.v.) and ibuprofen (10 mg/kg, i.v.) treatments on nociceptive parameters 24h post-CYP injection. Nociceptive threshold (A), nociceptive scores (B), AUC 1-8 g (C) or AUC 8-60 g (D) 24h after CYP injection. Results are expressed as mean ± s.e.m. (n=10). 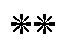 p<0.01, 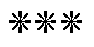 p<0.001 *vs* Vehicle-treated group, Mann Whitney test (A), Two-way RM ANOVA (B), Mann Whitney test and Unpaired *t* test (C) and Unpaired *t* test (D).

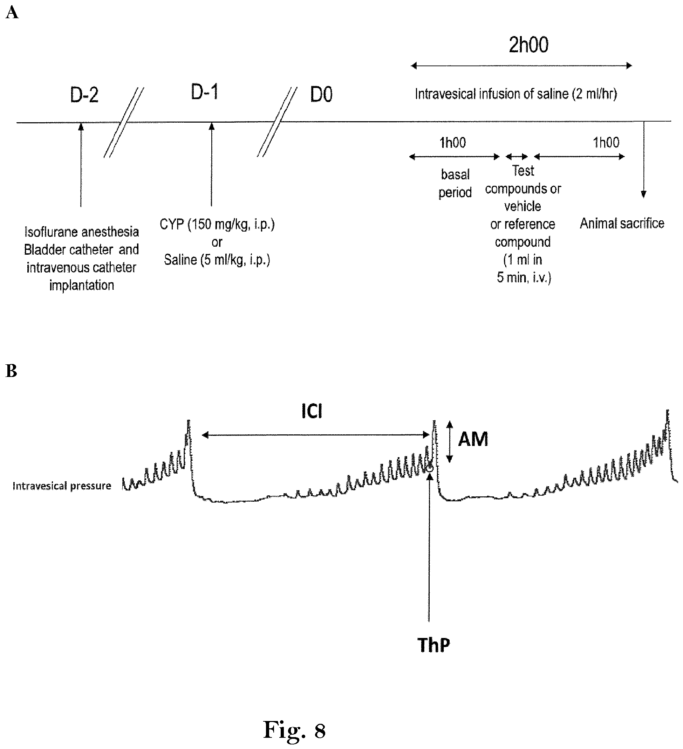

- Figure 8: shows for Example 13 the study design (A) and the cystometric parameters analysed (B).

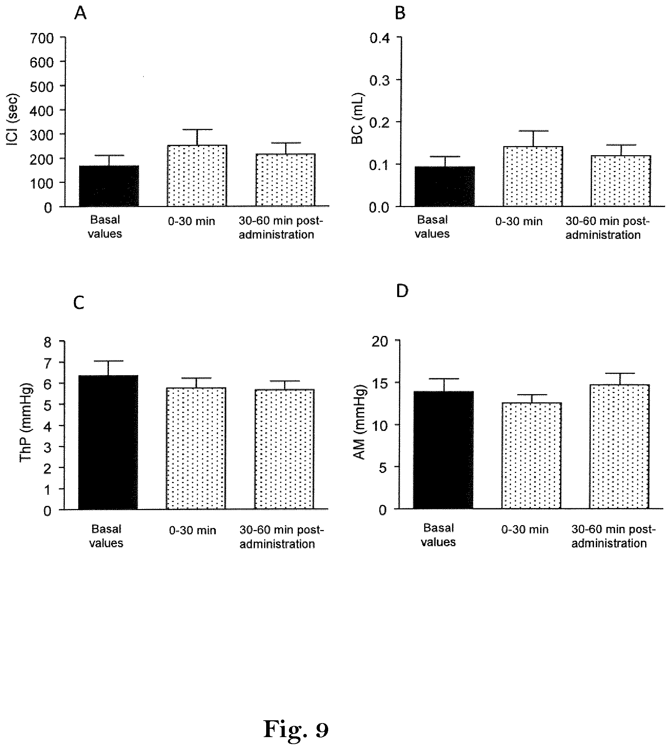

- Figure 9: 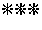 shows for Example 13 the effects of vehicle (i.v.) on cystometric parameters in conscious female rats treated with CYP. Not significant *versus* basal values with a one way ANOVA with repeated measures, followed by a Dunnett's post-test.

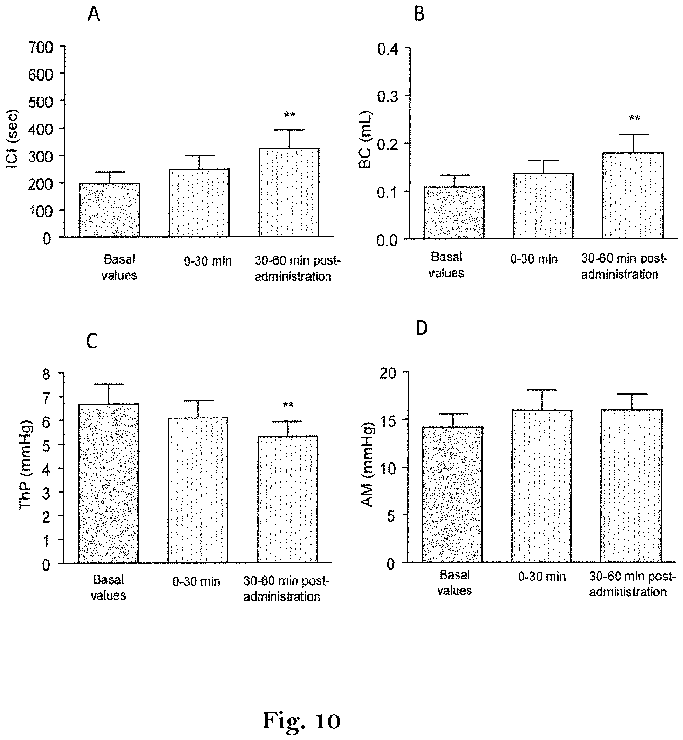

- Figure 10: 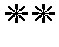 shows for Example 13 the effects of XG-102 (2 mg/kg, i.v.) on cystometric parameters in conscious female rats treated with CYP. 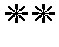 P< 0.01 *versus* basal values with a one way ANOVA with repeated measures, followed by a Dunnett's post-test.

### EXAMPLES

### Example 1: Identification of JNK Inhibitor sequences

Amino acid sequences important for efficient interaction with JNK were identified by sequence alignments between known JNK binding domain JBDs. A sequence comparison between the JBDs of IB1 [SEQ ID NO: 13], IB2 [SEQ ID NO: 14], c-Jun [SEQ ID NO: 15] and ATF2 [SEQ ID NO: 16] defined a weakly conserved 8 amino acid sequence (see Figure 1A). Since the JBDs of IB1 and IB2 are approximately 100 fold as efficient as c-Jun or ATF2 in binding JNK (Dickens et al. Science 277: 693 (1997), it was reasoned that conserved residues between IB1 and IB2 must be important to confer maximal binding. The comparison between the JBDs of IB1 and IB2 defined two blocks of seven and three amino acids that are highly conserved between the two sequences.

These two blocks are contained within a peptide sequence of 19 amino acids in L-IB1(s) [SEQ ID NO: 1] and are also shown for comparative reasons in a 23 aa peptide sequence derived from IB1 [SEQ ID NO: 17]. These sequences are shown in Figure 1B, dashes in the L-IB1 sequence indicate a gap in the sequence in order to align the conserved residues with L-IB1(s).

### Example 2: Preparation of JNK Inhibitor Fusion Proteins

JNK inhibitor fusion proteins according to SEQ ID NO: 9 were synthesized by covalently linking the C-terminal end of SEQ ID NO: 1 to a N-terminal 10 amino acid long carrier peptide derived from the HIV-TAT4g 57 (Vives et al., J Biol. Chem. 272: 16010 (1997)) according to SEQ ID NO: 5 via a linker consisting of two proline residues. This linker was used to allow for maximal flexibility and prevent unwanted secondary structural changes. The basic constructs were also prepared and designated L-IB1(s) (SEQ ID NO: 1) and L-TAT [SEQ ID NO: 5], respectively.

All-D retro-inverso peptides according to SEQ ID NO: 11 were synthesized accordingly. The basic constructs were also prepared and designated D-IB1(s) [SEQ ID NO: 2] and D-TAT [SEQ ID NO: 6], respectively.

All D and L fusion peptides according to SEQ ID NOs: 9, 10, 11 and 12 were produced by classical Fmock synthesis and further analysed by Mass Spectrometry. They were finally purified by HPLC. To determine the effects of the proline linker, two types of TAT peptide were produced one with and one without two prolines. The addition of the two prolines did not appear to modify the entry or the localization of the TAT peptide inside cells. Generic peptides showing the conserved amino acid residues are given in Figure 2.

### Example 3: Inhibition of Cell Death By JBD 19

Effects of the 19 aa long JBD sequence of IB1(s) on JNK biological activities were studied. The 19 aa sequence was linked N-terminal to the Green Fluorescent Protein (GFP JBD19 construct), and the effect of this construct on pancreatic⊐beta-cell apoptosis induced by IL1 was evaluated. This mode of apoptosis was previously shown to be blocked by transfection with JBD₁₋₂₈₀ whereas specific inhibitors of ERK1/2 or p38 as known in the art did not protect.

Oligonucleotides corresponding to JBD19 and comprising a conserved sequence of 19 amino acids as well as a sequence mutated at the fully conserved regions were synthesized and directionally inserted into the EcoRI and SalI sites of the pEGFP-N1 vector encoding the Green Fluorescent Protein (GFP) (from Clontech). Insulin producing ETC-3 cells were cultured in RPMI 1640 medium supplemented with 10% Fetal Calf Serum, 100 µg/mL Streptomycin, 100 units/mL Penicillin and 2 mM Glutamine. Insulin producing ETC-3 cells were transfected with the indicated vectors and IL-1⊏ (10 ng/mL) was added to the cell culture medium. The number of apoptotic cells was counted at 48 hours after the addition of IL-1⊐ using an inverted fluorescence microscope. Apoptotic cells were discriminated from normal cells by the characteristic "blebbing out" of the cytoplasm and were counted after two days.

GFP is Green Fluorescent protein expression vector used as a control; JBD19 is the vector expressing a chimeric GFP linked to the 19 aa sequence derived from the JBD of IB1; JBD19Mut is the same vector as GFP-JBD19, but with a JBD mutated at four conserved residues shown as Figure 1B ; and JBD₁₋₂₈₀ is the GFP vector linked to the entire JBD (aa 1-280). The GFP-JBD19 expressing construct prevented IL-1⊏⊏ induced pancreatic ⊐⊏-cell apoptosis as efficiently as the entire JBD₁₋₂₈₀.

As additional controls, sequences mutated at fully conserved IB1(s) residues had greatly decreased ability to prevent apoptosis.

### Example 4 : Cellular Import of TAT-IB1(s) Peptides

The ability of the L-and D-enantiomeric forms of TAT and TAT-IB1(s) peptides ("TAT-IB peptides") to enter cells was evaluated. L-TAT, D-TAT, L-TAT-IB1(s), and D-TAT-IB1(s) peptides [SEQ ID NOs: 5, 6, 9 and 12, respectively] were labeled by N-terminal addition of a glycine residue conjugated to fluorescein. Labeled peptides (1 µM) were added to ⊏TC-3 cell cultures, which were maintained as described in Example 3. At predetermined times cells were washed with PBS and fixed for five minutes in ice-cold methanol-acetone (1:1) before being examined under a fluorescence microscope. Fluorescein-labeled BSA (1 µM, 12 moles/mole BSA) was used as a control. Results demonstrated that all the above fluorescein labeled peptides had efficiently and rapidly (less than five minutes) entered cells once added to the culture medium. Conversely, fluorescein labeled bovine serum albumin (1 µM BSA, 12 moles fluorescein/mole BSA) did not enter the cells.

A time course study indicated that the intensity of the fluorescent signal for the L-enantiomeric peptides decreased by 70% following a 24 hours period. Little to no signal was present at 48 hours. In contrast, D-TAT and D-TAT-IB1(s) were extremely stable inside the cells. Fluorescent signals from these all-D retro-inverso peptides were still very strong 1 week later, and the signal was only slightly diminished at 2 weeks post treatment.

### Example 5 : In vitro Inhibition of c-JUN, ATF2 and Elk1 Phosphorylation

The effects of the peptides on JNKs-mediated phosphorylation of their target transcription factors were investigated *in vitro.* Recombinant and non activated JNK1, JNK2 and JNK3 were produced using a TRANSCRIPTION AND TRANSLATION rabbit reticulocyte lysate kit (Promega) and used in solid phase kinase assays with c-Jun, ATF2 and Elk1, either alone or fused to glutathione-S-transferase (GST), as substrates. Dose response studies were performed wherein L-TAT or L-TAT-IB1(s) peptides (0-25 µM) were mixed with the recombinant JNK1, JNK2, or JNK3 kinases in reaction buffer (20 mM Tris-acetate,1mM EGTA, 10 mM p-nitrophenyl-phosphate (pNPP), 5 mM sodium pyrophosphate, 10 mM p-glycerophosphate,1 mM dithiothreitol) for 20 minutes. The kinase reactions were then initiated by the addition of 10 mM MgCl₂ and 5 pCi ³³P-gamma-dATP and 1 µg of either GST-Jun (aa 1-89), GST-AFT2 (aa 1-96) or GST-ELK1 (aa 307-428). GST-fusion proteins were purchased from Stratagene (La Jolla, CA).

Ten µL of glutathione-agarose beads were also added to the mixture. Reaction products were then separated by SDS-PAGE on a denaturing 10 % polyacrylamide gel. Gels were dried and subsequently exposed to X-ray films (Kodak). Nearly complete inhibition of c-Jun, ATF2 and Elk1 phosphorylation by JNKs was observed at TAT-IB(s) peptide doses as low as 2.5 µM. However, a marked exception was the absence of TAT-IB(s) inhibition of JNK3 phosphorylation of Elk1. Overall, the TAT-IB1(s) peptide showed superior effects in inhibiting JNK family phosphorylation of their target transcription factors. The ability of D-TAT, D-TAT-IB1(s) and L-TAT-IB1(s) peptides (0-250 µM dosage study) to inhibit GST-Jun (aa 1-73) phosphorylation by recombinant JNK1, JNK2, and JNK3 by were analyzed as described above. Overall, D-TAT-IB1(s) peptide decreased JNK-mediated phosphorylation of c-Jun, but at levels approximately 10-20 fold less efficiently than L-TAT-IB1(s).

### Example 6: In vivo inhibition of c-JUN phosphorylation by TAT-IB(s) peptides as defined herein

To determine whether the cell-permeable peptides as defined herein could block JNK signaling *in vivo,* we used a heterologous GAL4 system. HeLa cells, cultured as described above, were co-transfected with the 5xGAL-LUC reporter vector together with the GAL-Jun expression construct (Stratagene) comprising the activation domain of c-Jun (amino acids 1-89) linked to the GAL4 DNA-binding domain. Activation of JNK was achieved by the co-transfection of vectors expressing the directly upstream kinases MKK4 and MKK7 (see Whitmarsh et al., Science 285: 1573 (1999)). Briefly, 3×10⁵ cells were transfected with the plasmids in 3.5-cm dishes using DOTAP (Boehringer Mannheim) following instructions from the manufacturer. For experiments involving GAL-Jun, 20 ng of the plasmid was transfected with1 µg of the reporter plasmid pFR-Luc (Stratagene) and 0.5 µg of either MKK4 or MKK7 expressing plasmids. Three hours following transfection, cell media were changed and TAT and TAT-IB1(s) peptides (1 µM) were added. The luciferase activities were measured 16 hours later using the "Dual Reporter System" from Promega after normalization to protein content. Addition of TAT-IB1(s) peptide blocked activation of c-Jun following MKK4 and MKK7 mediated activation of JNK. Because HeLa cells express JNK1 and JNK2 isoforms but not JNK3, we transfected cells with JNK3. Again, the TAT-IB(s) peptide inhibited JNK2 mediated activation of c-Jun.

### Example 7: Synthesis of all-D retro-inverso IB(s) Peptides and variants thereof

Peptides of the invention may be all-D amino acid peptides synthesized in reverse to prevent natural proteolysis (i.e. all-D retro-inverso peptides). An all-D retro-inverso peptide of the invention would provide a peptide with functional properties similar to the native peptide, wherein the side groups of the component amino acids would correspond to the native peptide alignment, but would retain a protease resistant backbone.

Retro-inverso peptides of the invention are analogs synthesized using D-amino acids by attaching the amino acids in a peptide chain such that the sequence of amino acids in the retro-inverso peptide analog is exactly opposite of that in the selected peptide which serves as the model. To illustrate, if the naturally occurring TAT protein (formed of L-amino acids) has the sequence GRKKRRQRRR [SEQ ID NO: 5], the retro-inverso peptide analog of this peptide (formed of D-amino acids) would have the sequence RRRQRRKKRG [SEQ ID NO: 6]. The procedures for synthesizing a chain of D-amino acids to form the retro-inverso peptides are known in the art (see e.g. Jameson et al., Nature, 368,744-746 (1994); Brady et al., Nature, 368,692-693 (1994); Guichard et al., J. Med. Chem. 39,2030-2039 (1996)). Specifically, the retro-peptides according to SEQ ID NOs 2, 4, 6, 8, 11-12, 18, 20, 22 and 25-26, were produced by classical F-mock synthesis and further analyzed by Mass Spectrometry. They were finally purified by HPLC.

Since an inherent problem with native peptides is degradation by natural proteases and inherent immunogenicity, the heterobivalent or heteromultivalent compounds of this invention will be prepared to include the "retro-inverso isomer" of the desired peptide. Protecting the peptide from natural proteolysis should therefore increase the effectiveness of the specific heterobivalent or heteromultivalent compound, both by prolonging half-life and decreasing the extent of the immune response aimed at actively destroying the peptides.

### Example 8: Long term biological activity of all-D retro-inverso IB(s) Peptides and variants thereof

Long term biological activity is predicted for the D-TAT-IB(s) retro-inverso containing peptide heteroconjugate (see chimeric sequences above) when compared to the native L-amino acid analog owing to protection of the D-TAT-IB(s) peptide from degradation by native proteases, as shown in Example 5.

Inhibition of IL-1⊏⊐ induced pancreatic beta-cell death by the D-TAT-IB1(s) peptide was analyzed. ⊏TC-3 cells were incubated as described above for 30 minutes with one single addition of the indicated peptides (1 µM), then IL-1 (10 ng/ml) was added.

Apoptotic cells were then counted after two days of incubation with IL-1⊐⊏ by use of Propidium Iodide and Hoechst 33342 nuclear staining. A minimum of 1,000 cells were counted for each experiment. Standard Error of the Means (SEM) are indicated, n=5. The D-TAT-IB1 peptide decreased IL-1 induced apoptosis to a similar extent as L-TAT-IB peptides.

Long term inhibition of IL-1P induced cell-death by the D-TAT-IB1 peptide was also analyzed. ⊏TC-3 cells were incubated as above for 30 minutes with one single addition of the indicated peptides (1 µM), then IL-1⊐⊏ C (10 ng/ml) was added, followed by addition of the cytokine every two days. Apoptotic cells were then counted after 15 days of incubation with IL-1 by use of propidium iodide and Hoechst 33342 nuclear staining. Note that one single addition of the TAT-IB1 peptide does not confer long-term protection. A minimum of 1.000 cells were counted for each experiment. As a result, D-TAT-IB1(s), but not L-TAT-IB1(s), was able to confer long term (15 day) protection.

### Example 9: Inhibition of endogenous JNK activity in HepG2 cells using an all-in one well approach (see Figure 3).

HepG2 cells were seeded at 3'000 cells/well the day prior the experiment. Then, increasing concentrations of either interleukin-1⊐ [IL-1beta⊐v)] or tumor necrosis factor ⊐ [TNFalpha] (a) were added to activate JNK for 30 minutes. Cells were lysed in 20mM Hepes, 0.5% Tween pH 7.4 and processed for AlphaScreen JNK. (b) Z' for the JNK activity induced by 10 ng/ml IL-1⊐ and measured in 384 wells/plate (n=96). (c) Inhibition of endogenous IL-1beta-induced JNK activity with chemical JNK inhibitors [staurosporin and SP600125]. (d) Effect of peptidic inhibitors L-TAT-IB1(s) according to SEQ ID NO: 9 [here abbreviated as L-JNKi (v)) and D-TAT-IB1(s) according to SEQ ID NO: 11 (here abbreviated as D-JNKi) and JBDs (corresponds to L-JNKI without the TAT sequence)] on IL-1⊐ dependent JNK activity. All panels are representative of three independent experiments (n=3).

### Methods: Alphascreen kinase assay

Principle: AlphaScreen is a non-radioactive bead-based technology used to study biomolecular interactions in a microplate format. The acronym ALPHA stands for Amplified Luminescence Proximity Homogenous Assay. It involves a biological interaction that brings a "donor" and an "acceptor" beads in close proximity, then a cascade of chemical reactions acts to produce an amplified signal. Upon laser excitation at 680 nm, a photosensitizer (phthalocyanine) in the "donor" bead converts ambient oxygen to an excited singlet state. Within its 4 µsec half-life, the singlet oxygen molecule can diffuse up to approximately 200 nm in solution and if an acceptor bead is within that proximity, the singlet oxygen reacts with a thioxene derivative in the "acceptor" bead, generating chemiluminescence at 370 nm that further activates fluorophores contained in the same "acceptor" bead. The excited fluorophores subsequently emit light at 520-620 nm. In the absence of an acceptor bead, singlet oxygen falls to ground state and no signal is produced.

Kinase reagents (B-GST-cJun, anti P-cJun antibody and active JNK3) were first diluted in kinase buffer (20 mM Tris-HCl pH 7.6, 10 mM MgCl₂, 1 mM DTT, 100 µM Na₃VO₄, 0.01% Tween-20) and added to wells (15 µl). Reactions were then incubated in presence of 10 µM of ATP for 1h at 23°C. Detection was performed by an addition of 10 µl of beads mix (Protein A acceptor 20 µg/ml and Streptavidin donor 20 µg/ml), diluted in detection buffer (20 mM Tris-HCl pH 7.4, 20 mM NaCl, 80 mM EDTA, 0.3% BSA), followed by an another one-hour incubation at 23°C in the dark. For measurement of JNK endogenous activity, kinase assays were performed as described above except active JNK3 was replaced by cells lysates and reaction kinase components were added after the cells lysis. B-GST-cjun and P-cJun antibody were used at the same concentrations whereas ATP was used at 50 µM instead of 10 µM. AlphaScreen signal was analyzed directly on the Fusion or En Vision apparatus.

### Example 10: Safety, tolerability and pharmacokinetics of a single intravenous infusion of 10, 40 and 80 µg/kg XG-102 (SEQ ID No.: 11) administered to healthy male volunteers in a randomized, double blind, placebo controlled, dose escalating Phase I study

The primary objective of the study was to assess the safety and tolerability of XG-102 following intravenous (iv) infusion of single escalating doses of XG-102 to healthy male volunteers. The secondary objective of the study was to assess the pharmacokinetics of XG-102 following iv infusion of single escalating doses of XG-102 to healthy male volunteers. Doses were administered as a 60 minute iv infusion. For control purposes, placebo iv infusion was administered to control subjects.

This was a single-centre, randomized, double blind, placebo controlled, ascending single dose, sequential group study. Three dose levels of XG-102 (10, 40 and 80 µg/kg) were studied in ascending order of dose, within each group subjects were randomized such that 6 subjects received XG-102, and 2 subjects received placebo. Screening was performed in the 3-week period prior to dosing. Dosing occurred on Day 0 for each subject. The Investigator checked on all subjects' well-being prior to their discharge from the CRU (at 24 hours after dosing). Subjects returned to the CRU 8 ±2 days and 28 ±5 days after dosing for post study assessments.

A total of 24 subjects (healthy male subjects in the age of 18 to 45), in 3 groups of 8. 24 subjects entered and completed the study. Data for all subjects were included in the safety analyses; data for all subjects who received XG-102 were included in the pharmacokinetic analyses.

| *Summary:* | | | | |
|---|---|---|---|---|
| *Pharmacokinetic results:* | | | | |
| The pharmacokinetic parameters of XG-102 are presented in the following table: | | | | |
| | Parameter | 10 µg/kg (N=6) | 40 µg/kg (N=6) | 80 µg/kg (N=6) |
| | AUC0₀₋ₗₐₛₜ (ng.h/mL) | 24.7 (26.1) | 134 (15.2) | 431 (41.0) |
| | AUC_{0-∞} (ng.h/mL) | 36.8 (23.4) | 146 (17.5) | 443 (41.0) |
| | AUCextrap^{a} | 34.1 (18.6 - 49.7) | 6.7 (4.2 - 12.9) | 2.9 (1.9 - 3.4) |
| | Cₘₐₓ (ng/mL) | 31.3 (24.4) | 146 (16.7) | 362 (34.9) |
| | tₘₐₓ^{a} (h) | 1.00 (1.00 - 1.05) | 1.00 (1.00 - 1.00) | 1.00 (1.00 - 1.00) |
| | AUC₀₋ₗₐₛₜ(norm) (ng.h/mL)/(µg/kg) | 3.10 (29.3) | 3.64 (13.8) | 5.91 (41.8) |
| | AUC_{0-∞}(norm) (ng.h/mL)/(µg/kg) | 4.61 (24.8) | 3.96 (15.7) | 6.07 (41.8) |
| | Cₘₐₓ(norm) (ng/mL)/(µg/kg) | 3.93 (28.0) | 3.98 (15.9) | 4.97 (35.6) |
| | MRT (h) | 1.00 (29.9) | 0.76 (11.0) | 1.02 (14.7) |
| | t_{½} (h) | 0.57 (44.6) | 0.36 (22.3) | 0.65 (38.8) |
| | CL (mL/h) | 17537 (23.9) | 18399 (16.4) | 13217 (43.5) |
| | CL (mL/h/kg) | 217 (24.8) | 253 (15.7) | 165 (41.8) |
| | Vₛₛ (mL) | 17536 (36.8) | 14040 (15.7) | 13500 (30.5) |
| | Vₛₛ (mL/kg) | 217 (27.5) | 193 (13.7) | 168 (29.8) |
| | Geometric mean (CV%) data are presented | | | |
| | N = Number of subjects studied | | | |
| | (norm) = Normalized for dose and body weight | | | |
| | a Median (min max) | | | |

The observed values of t_{1/2} were short. Both peak exposure as measured by Cₘₐₓ and cumulative exposure as measured by AUC₀₋ₗₐₛₜ increased with dose. The increase with dose of Cₘₐₓ appears to be more than linearly proportional on the basis of graphical examinations and of the geometric mean of its dose normalized values which after the highest 80 µg/kg dose are above the 90% confidence intervals for the other doses. The increase with dose of AUC₀₋ₗₐₛₜ is clearly more than linearly proportional from 40 to 80 µg/kg as the 90% confidence intervals for its geometric mean dose normalized value does not overlap with those after the other tested doses; whereas when comparing values after 10 and 40 µg/kg the 90% confidence intervals overlap, but its geometric mean dose normalized value after the 10 µg/kg dose is lower than all values in the corresponding 90% confidence interval after the 40 µg/kg dose.

XG-102 was safe and well tolerated when administered as single iv doses of 10, 40 or 80 µg/kg to healthy male subjects. The incidence of adverse events in subjects who received XG-102 was similar to the incidence in subjects who received placebo. There were no clinically significant findings in clinical laboratory data, vital signs, ECGs, physical examinations or ocular examinations (fundus and IOP).

After the end of XG-102 intravenous infusion, its plasma concentrations quickly decreased, leading to values below the lower limit of quantification by at most 2 hours after the start of 10 µg/kg XG-102 iv infusions, 3 hours after the start of 40 µg/kg XG-102 iv infusions and by at most 7 hours after the start of 80 µg/kg XG-102 intravenous infusions. The measured t_{1/2} and MRT values are short, with geometric mean values per dose level ranging from 0.36 to 0.65 hours and from 0.76 to 1.02 hours, respectively.

The AUC₀₋ₗₐₛₜ of XG-102 increases in a more than linear proportion with dose in the tested dose range, with non-overlapping 90% confidence intervals for its geometric mean dose normalized values between the 40 µg/kg and the 80 µg/kg dose and only limited overlap between the 90% confidence intervals for its geometric mean dose normalized values between the 10 µg/kg and the 40 µg/kg.

The Cₘₐₓ of XG-102 appears to increase in a more than linear proportion with dose from 40 to 80 µg/kg. The geometric mean dose normalized Cₘₐₓ in the 80 µg/kg dose group is higher than and outside the 90% confidence intervals for the geometric mean dose normalized Cₘₐₓ in the other dose groups, but the 90% confidence intervals for the geometric mean dose normalized Cₘₐₓ overlap among all dose levels.

The intersubject variability of XG-102 pharmacokinetic parameters was moderate in subjects treated with 10 and 40 µg/kg doses (CV% of the geometric mean for most parameters approximately in the 15-30% range, exception was t_{1/2} and total Vₛₛ at the 10 µg/kg dose group), but higher in the 80 µg/kg dose group, in the approximately 29-44% range, other than for MRT (14.7%). This higher variability may be either an effect of the low sample size or a consequence of the observed non-linearities which are clearer at this dose.

### Example 11: Effects of XG-102 (SEQ ID No. 11) administered intravesically on acute cystitis model induced by cyclophosphamide in conscious rats: Evaluation of visceral pain and urinary bladder inflammation

The aim of the present study was to evaluate the effects of intravesical treatment with XG-102 (50 mg/mL) on urinary bladder pain and inflammation in acute CYP-induced cystitis in female Sprague-Dawley rats. This preclinical model is well-used to test therapeutic approaches for the treatment of interstitial cystitis / painful bladder syndrome (IC/PBS).

Adult female Sprague-Dawley rats (Janvier Labs, Le Genest Saint Isle, France), weighing 215 ± 20 g at the beginning of the experiments, were used. Animals were acclimatized to the laboratory conditions for at least 3 days before the start of any experiments. The animals were allocated to the following four experimental groups (n=10 animals per group):

| **Group** | **Injection (i.p.)** | **Treatment (i.ves.)** | **n** |
|---|---|---|---|
| 1 | Saline | Vehicle (500 µL, i.ves.) | 10 |
| 2 | CYP | Vehicle (500 µL, i.ves.) | 10 |
| 3 | CYP | XG-102 (50 mg/mL, i.ves.) | 10 |
| 4 | CYP | Ibuprofen (50 mg/mL, i.ves.) | 10 |

To induce acute cystitis, a single i.p. injection of CYP at a dose of 150 mg/kg in a final volume of 5 mL/kg was performed. Control rats received physiological saline under the same experimental conditions as CYP (final volume of 5 mL/kg, i.p.).

On the day of each experiment, weight of rats was recorded. Then, in a randomized manner, 500 µL of XG-102 (50 mg/mL), ibuprofen (50 mg/mL) or vehicle were intravesically infused during 30 min under isoflurane anesthesia (2% - 3%).

Assessment of referred visceral pain using von Frey filaments:
Standardized conditions including fixed time-of-day (a.m. to minimize the potential circadian variations in the behaviours responses) and single-experimenter testing of all animals were applied to minimize variability behavior-based pain testing. Visceral pain including allodynia and hyperalgesia was evaluated by applying to the lower abdomen, close to the urinary bladder, a set of 8 calibrated von Frey filaments of increasing forces (1, 2, 4, 6, 8, 10, 26 and 60 g) with an interstimulus interval of 5 seconds. Prior testing, the abdominal area designed for mechanical stimulation of each animal was shaved. Animals were then placed on a raised wire mesh floor under individual transparent Plexiglas box and acclimatized for at least 30 minutes before starting the von Frey test. Filaments were then applied 1-2 seconds through the mesh floor with enough strength to cause the filament to slightly bend. Each filament was tested 3 times. Care was taken to stimulate different areas within the lower abdominal region in the vicinity of the urinary bladder to avoid desensitization.

Nociceptive behaviors were scored for each animal and each filament as follows:

| **Score** | **Behavior** |
|---|---|
| 0 | no response |
| 1 | reaction of the animal (e.g. retraction of the abdomen) |
| 2 | reaction of the animal and change of position |
| 3 | reaction of the animal, change of position and licking of the site stimulated with von Frey filaments and / or vocalization |

The study design is schematically shown in Fig. 4 A. Birefly, acute cystitis was induced by CYP injection (i.p.) at D0 (as described above). XG-102, ibuprofen or vehicle was intravesically administrated once just after CYP injection (as described above). Von Frey testing was performed in a non-blinded manner as follow:
- At D-1, rats were acclimatized to the individual Plexiglas box for a minimum of 30 min and to the von Frey filaments application, in order to decrease the level of stress due to the new environment.
- At D0, von Frey testing was performed 15 min before CYP or saline injection in order to obtain basal values (D0, T=-15min).
- At D1, von Frey testing was performed 24 hours after CYP or saline injection in order to analyze test compounds effect on CYP-induced visceral pain (D1, T=+24h).
- Just after von Frey testing (+24h), rats were anesthetized for blood samples collection, then sacrificed and urinary bladders were collected as described below.

At the end of the experiment, rats were sacrificed by injection of pentobarbital (54.7 mg/mL, 0.5 mL/rat, i.p.) followed by cervical dislocation. Urinary bladders were rapidly collected and cleaned from lipoid tissue. Urinary bladders were weighed, cut at the bladder neck and haemorrhage scoring was performed (see table below). Finally, wall thickness was measured using a digital caliper by placing the bladder wall between the two outside jaws. Urinary bladder haemorrhage scores were adapted from Gray's criteria (Gray *et al.*, 1986) as follows:

| **Scores** | **Haemorrhage** |
|---|---|
| 0 | absent - normal aspect |
| 1 | telangiectasia - dilatation of the mucosal blood vessels |
| 2 | petechial haemorrhages - mucosal pinpoint red dots (glomerulation) |
| 3 | Hemorrhagic spots with blood clots |

Nociceptive parameters are expressed as follows:

| **Parameters** | **Expression** | **Description** |
|---|---|---|
| nociceptive threshold | g | von Frey filament for which a first score of at least 1 (for 3 applications) is obtained |
| nociceptive scores | % | % of the maximal response (maximum score = 9) for 3 pooled applications |
| area under the curve (AUC) 1-8 g (allodynia) | % scores x | plot of individual percentage of nociceptive scores against von Frey forces from : 1 to 8 g or 8 to 60g |
| area under the curve (AUC) 8-60 g (hyperalgesia) | g | |

AUCs were calculated using GraphPad Prism^{®} (GraphPad Software Inc., La Jolla, CA, USA). The AUCs method to assess allodynia and hyperalgesia is schematically shown in Figure 4 B.

Macroscopic parameters are expressed as follows:

| **Parameters** | **Expression** |
|---|---|
| whole urinary bladder weight | mg and % of body weight |
| haemorrhage | scores |
| urinary wall thickness | mm |

### Results:

Before CYP injection, no significant difference in the nociceptive parameters were observed between the 3 different CYP-injected groups. In order to analyse effect of XG-102 on CYP-induced visceral pain, nociceptive parameters were compared between the Vehicle- and the XG-102-treated groups. Twenty-four hours after CYP injection, nociceptive threshold was significantly increased by XG-102 treatment as compared to vehicle (p<0.01, Figure 5 A). XG-102 treatment also significantly decreased nociceptive scores in CYP-injected rats as compared to vehicle (p<0.001, Figure 5 B). In addition, AUC 1-8 g was significantly decreased by XG-102 treatment as compared to vehicle (p<0.001, Figure 5 C). Similarly, AUC 8-60 g was reduced by XG-102 treatment as compared to vehicle (p<0.01, Figure 5 D). In order to analyse the effects of ibuprofen on CYP-induced visceral pain, nociceptive parameters were compared between the Vehicle- and the Ibuprofen-treated groups. Nociceptive threshold was significantly increased by ibuprofen treatment as compared to vehicle in CYPinjected rats (p<0.01, Figure 5 A). Similarly in the Ibuprofen group significant decrease of nociceptive scores was observed as compared to vehicle (p<0.01, Figure 5 B). In addition, AUC 1-8 g and AUC 8-60 g were significantly decreased by ibuprofen treatment as compared to vehicle (p<0.001 and p<0.05, Figures 5 C and 101 D, respectively).

Moreover, urinary wall thickness was significantly decreased in XG-102-treated rats (p<0.01, Figure 6 A). Although XG-102 treatment also showed a trend towards decreased haemorrhage scores, it did not reach statistical significance (Figure 6 B). For ibuprofen, also a significant decrease was observed in urinary bladder wall thickness (p<0.001, Figure 6 A). However, no significant change was observed regarding haemorrhage scores (p>0.05, Figure 6 B) in the Ibuprofen-treated group. It is noteworthy that reddish urine was noticed for some animal in the Ibuprofen-treated group.

Taken together, intravesical treatment of XG-102 (50 mg/mL) significantly reversed visceral pain induced by CYP, 24h after its injection. XG-102 efficiently inhibited both allodynia and hyperalgesia. On analyzed inflammatory parameters, XG-102 decreased urinary bladder inflammation (wall thickness). In conclusion, administered intravesically, XG-102 displayed strong antinociceptive effects and significant anti-inflammatory properties in an experimental model of IC/PBS.

### Example 12: Effects of XG-102 (SEQ ID No. 11) administered intravenously on acute cystitis model induced by cyclophosphamide in conscious rats: Evaluation of visceral pain (comparative)

The aim of the present study was to evaluate the effects of intravenous treatment with XG-102 (2 mg/kg) on urinary bladder pain in acute CYP-induced cystitis in female Sprague-Dawley rats. This preclinical model is well-used to test therapeutic approaches for the treatment of interstitial cystitis / painful bladder syndrome (IC/PBS).

Adult female Sprague-Dawley rats (Janvier Labs, Le Genest Saint Isle, France), weighing 215 ± 20 g at the beginning of the experiments, were used. Animals were acclimatized to the laboratory conditions for at least 3 days before the start of any experiments. The animals were allocated to the following four experimental groups (n=10 animals per group):

| **Group** | **Injection (i.p.)** | **Treatment (i.ves.)** | **n** |
|---|---|---|---|
| 1 | Saline | Vehicle (1 mL/kg, i.v.) | 10 |
| 2 | CYP | Vehicle (1 mL/kg, i.v.) | 10 |
| 3 | CYP | XG-102 (2 mg/kg, i.v.) | 10 |
| 4 | CYP | Ibuprofen (10 mg/kg, i.v.) | 10 |

To induce acute cystitis, a single i.p. injection of CYP at a dose of 150 mg/kg in a final volume of 5 mL/kg was performed. Control rats received physiological saline under the same experimental conditions as CYP (final volume of 5 mL/kg, i.p.).

On the day of each experiment, weight of rats was recorded. Then, in a randomized manner, XG-102 (2 mg/kg), ibuprofen (10 mg/kg) or vehicle were intravenously administered at a volume of 1 mL/kg.

Assessment of referred visceral pain using von Frey filaments:
Standardized conditions including fixed time-of-day (a.m. to minimize the potential circadian variations in the behaviours responses) and single-experimenter testing of all animals were applied to minimize variability behavior-based pain testing. Visceral pain including allodynia and hyperalgesia was evaluated by applying to the lower abdomen, close to the urinary bladder, a set of 8 calibrated von Frey filaments of increasing forces (1, 2, 4, 6, 8, 10, 26 and 60 g) with an interstimulus interval of 5 seconds. Prior testing, the abdominal area designed for mechanical stimulation of each animal was shaved. Animals were then placed on a raised wire mesh floor under individual transparent Plexiglas box and acclimatized for at least 30 minutes before starting the von Frey test. Filaments were then applied 1-2 seconds through the mesh floor with enough strength to cause the filament to slightly bend. Each filament was tested 3 times. Care was taken to stimulate different areas within the lower abdominal region in the vicinity of the urinary bladder to avoid desensitization.

Nociceptive behaviors were scored for each animal and each filament as follows:

| **Score** | **Behavior** |
|---|---|
| 0 | no response |
| 1 | reaction of the animal (*e.g*. retraction of the abdomen) |
| 2 | reaction of the animal and change of position |
| 3 | reaction of the animal, change of position and licking of the site stimulated with von Frey filaments and / or vocalization |

The study design differs from that of Example 11 (cf. Figure 4 A) only in the route of administration (intravenously instead of intravesically) and the doses as specified above. Birefly, acute cystitis was induced by CYP injection (i.p.) at D0 (as described above). XG-102, ibuprofen or vehicle was intravvenously administrated once just after CYP injection (as described above). Von Frey testing was performed in a non-blinded manner as follow:
- At D-1, rats were acclimatized to the individual Plexiglas box for a minimum of 30 min and to the von Frey filaments application, in order to decrease the level of stress due to the new environment.
- At D0, von Frey testing was performed 15 min before CYP or saline injection in order to obtain basal values (D0, T=-15min).
- At D1, von Frey testing was performed 24 hours after CYP or saline injection in order to analyze test compounds effect on CYP-induced visceral pain (D1, T=+24h).
- Just after von Frey testing (+24h), rats were anesthetized for blood samples collection, then sacrificed and urinary bladders were collected as described below.

Nociceptive parameters are expressed as follows:

| **Parameters** | **Expression** | **Description** |
|---|---|---|
| nociceptive threshold | g | von Frey filament for which a first score of at least 1 (for 3 applications) is obtained |
| nociceptive scores | % | % of the maximal response (maximum score = 9) for 3 pooled applications |
| area under the curve (AUC) 1-8 g (allodynia) | % scores x | plot of individual percentage of nociceptive scores against von Frey forces from : 1 to 8 g or 8 to 60g |
| area under the curve (AUC) 8-60 g (hyperalgesia) | g | |

AUCs were calculated using GraphPad Prism^{®} (GraphPad Software Inc., La Jolla, CA, USA). The AUCs method to assess allodynia and hyperalgesia is schematically shown in Figure 4 B.

### Results:

Before CYP injection, no significant difference in the nociceptive parameters was observed between the 3 different CYP-injected groups. In order to analyse the effect of XG-102 on CYP-induced visceral pain, nociceptive parameters were compared between the Vehicle- and the XG-102-treated groups independently. Twenty-four hours after CYP injection, nociceptive threshold was significantly increased by XG-102 treatment as compared to vehicle (p<0.01, Figure 7 A). XG-102 treatment significantly decreased nociceptive scores in CYP-injected rats as compared to vehicle (p<0.001, Figure 7 B). In addition, AUC 1-8 g was significantly decreased by XG-102 treatment as compared to vehicle (p<0.001, Figure 7 C). Similarly, AUC 8-60 g was significantly reduced by XG-102 treatment as compared to vehicle (p<0.001, Figure 7 D). In order to analyse effect of ibuprofen on CYP-induced visceral pain, nociceptive parameters were compared between Vehicle- and Ibuprofen-treated groups. Nociceptive threshold was significantly increased by ibuprofen treatment as compared to vehicle in CYPinjected rats (p<0.01, Figure 7 A). Ibuprofen treatment significantly decrease nociceptive scores as compared to vehicle (p<0.001, Figure 7 B).

In addition, AUC 1-8 g and AUC 8-60 g were significantly reduced by ibuprofen treatment as compared to vehicle (p<0.001, Figures 7 C and 7 D).

Taken together, intravenous treatment of XG-102 (2 mg/kg) thus significantly reversed visceral pain induced by CYP, 24h after its injection. XG-102 efficiently inhibited both allodynia and hyperalgesia. Similar effects were observed with intravenous administration of ibuprofen (10 mg/kg). In conclusion, in the experimental cystitis preclinical model, XG-102 displayed significant anti-nociceptive properties.

### Example 13: Effects of XG-102 (SEQ ID No. 11) administered intravenously on cystometric parameters in conscious rats with acute cystitis induced by cyclophosphamide (comparative)

The aim of the present study was to evaluate the effects of intravenous (i.v.) administration of XG-102 (2 mg/kg) on cystometric parameters in CYP-induced cystitis in conscious female Sprague-Dawley rats. This preclinical model is well-used to test therapeutic approaches for the treatment of interstitial cystitis / painful bladder syndrome (IC/PBS).

Female Sprague-Dawley rats (211 - 281 g) were used (Janvier Labs, Le Genest Saint Isle, France). They were delivered to the laboratory at least 5 days before the experiments in order to be acclimatized to laboratory conditions. The animals were allocated to the following three experimental groups:

| **Groups** | **i.p. administration** | **i.v. treatment** | **dose** | **n** |
|---|---|---|---|---|
| 1 | Physiological saline | Vehicle | - | 11 |
| 2 | CYP 150 mg/kg | Vehicle | - | 10 |
| 3 | CYP 150 mg/kg | XG-102 | 2 mg/kg | 11 |

Rats were anesthetized with isoflurane (1.5 - 3%). After a laparotomy, bladder was exteriorized and a polyethylene catheter (0.58 and 0.96 mm of internal and outer diameter, respectively) was implanted in the bladder through the dome and exteriorized at the scapular level. A jugular polyethylene catheter (0.58 and 0.96 mm of internal and outer diameter, respectively) was also implanted and exteriorized at the scapular level for i.v. administrations.

At D-1 (24 hours after the surgery), a single dose of CYP at 150 mg/kg or its vehicle (physiological saline: 0.9% NaCl) was administered i.p. at 5 mL/kg.

The method evaluating the effects of test substances on lower urinary tract function has been described by Lluel P, Barras M, Palea S. Cholinergic and purinergic contribution to the micturition reflex in conscious rats with long-term bladder outlet obstruction. Neurourol Urodyn. 2002; 21: 142-153. Cystometric investigations were performed in conscious rats 24 hours postintraperitoneal injection of CYP or vehicle. On the day of experiment, animals were held under partial restraint in a restraining device. The bladder catheter was connected via a T-tube to a pressure transducer to measure the intravesical pressure and to an injection pump to fill the bladder at a rate of 2 mL/hr. Vesical pressure was recorded continuously for 120 min: a 60 min as a basal period before intravenous administration and a 60 min period post-administration.

XG-102 or vehicle (1 mL in 5 min) was administered intravenously after 1 hour of basal period.

The study design is schematically shown in Fig. 8 A.

The following cystometric parameters were analysed (see Fig. 8 B):
- Threshold Pressure (ThP, mmHg), pressure just before micturition,
- Amplitude of micturition (AM), *i.e.* pressure between threshold pressure (ThP) and maximal pressure of micturition (MP) (mmHg),
- Intercontraction interval (ICI), *i.e.* time between two subsequent micturitions (sec), and
- Bladder capacity (BC), *i.e.* ICI x infusion rate (mL).

### Results:

No effects of vehicle (i.v.) was observed on the cystometric parameters ICI, BC, ThP and AM parameters in conscious rats treated with CYP, compared to basal values (Figure 9 A, B, C and D). In contrats, XG-102 (2 mg/kg, i.v.) significantly increased ICI and BC 30-60 min post-administration in CYP-treated rats, compared to basal values (P<0.01, Figure 10 A and B). This increase was associated with a significant decrease in ThP at the same time point (P<0.01, Figure 10 C).

Taken together, intravenous treatment of XG-102 (2 mg/kg) significantly increased ICI and BC and decreased ThP for the period of 30-60 min post administration.

## Claims

1. Chimeric peptide comprising at least one first domain and at least one second domain linked by a covalent bond, the first domain comprising a trafficking sequence, and the second domain comprising a JNK inhibitor sequence for use in the treatment of a disease or disorder strongly related to JNK signaling in a subject by intravesical administration, wherein the disease or disorder strongly related to JNK signaling in a subject is cystitis, in particular interstitial cystitis, and wherein the chimeric peptide consists of the amino acid sequence of SEQ ID NO: 11.

2. The chimeric peptide for use according to claim 1, wherein the JNK inhibitor sequence binds c-jun amino terminal kinase (JNK).

3. The chimeric peptide for use according to any of claims 1 to 2, wherein the JNK inhibitor sequence inhibits the activation of at least one JNK targeted transcription factor when the JNK inhibitor sequence is present in a JNK expressing cell.

4. The chimeric peptide for use according to any of claims 1 to 3, wherein the JNK targeted transcription factor is selected from the group consisting of c-Jun, ATF2, and Elkl.

5. The chimeric peptide for use according to any of claims 1 to 4, wherein the JNK inhibitor sequence alters a JNK effect when the peptide is present in a JNK expressing cell.

6. The chimeric peptide for use according to any of claims 1-5, wherein the trafficking sequences augments cellular uptake of the peptide.

7. The chimeric peptide for use according to any of claims 1-6, wherein the trafficking sequence directs nuclear localization of the peptide.

## Patentansprüche

1. Chimäres Peptid, umfassend mindestens eine erste Domäne und mindestens eine zweite Domäne, die durch eine kovalente Bindung verbunden sind, wobei die erste Domäne eine Transport-Sequenz umfasst und die zweite Domäne eine JNK-Inhibitorsequenz umfasst, zur Verwendung bei der Behandlung einer Krankheit oder Störung, die stark mit der JNK-Signalgebung in einem Subjekt verbunden ist, durch intravesikale Verabreichung, wobei die Krankheit oder Störung, die stark mit der JNK-Signalgebung in einem Subjekt verbunden ist, Zystitis, insbesondere interstitielle Zystitis, ist und wobei das chimäre Peptid aus der Aminosäuresequenz von SEQ ID NO: 11 besteht.

2. Chimäres Peptid zur Verwendung gemäß Anspruch 1, wobei die JNK-Inhibitorsequenz c-Jun aminoterminale Kinase (JNK) bindet.

3. Chimäres Peptid zur Verwendung nach einem der Ansprüche 1 bis 2, wobei die JNK-Inhibitorsequenz die Aktivierung mindestens eines JNK-gerichteten Transkriptionsfaktors hemmt, wenn die JNK-Inhibitorsequenz in einer JNK-exprimierenden Zelle vorhanden ist.

4. Chimäres Peptid zur Verwendung nach einem der Ansprüche 1 bis 3, wobei der JNK-gerichtete Transkriptionsfaktor ausgewählt ist aus der Gruppe bestehend aus c-Jun, ATF2 und Elk1.

5. Chimäres Peptid zur Verwendung nach einem der Ansprüche 1 bis 4, wobei die JNK-Inhibitorsequenz eine JNK-Wirkung verändert, wenn das Peptid in einer JNK-exprimierenden Zelle vorhanden ist.

6. Chimäres Peptid zur Verwendung nach einem der Ansprüche 1-5, wobei die Transport-Sequenzen eine zelluläre Aufnahme des Peptids erhöhen.

7. Chimäres Peptid zur Verwendung nach einem der Ansprüche 1-6, wobei die Transport-Sequenz eine Kernlokalisierung des Peptids steuert.

## Revendications

1. Peptide chimérique comprenant au moins un premier domaine et au moins un second domaine liés par une liaison covalente, le premier domaine comprenant une séquence de trafic, et le second domaine comprenant une séquence inhibitrice de JNK destinée à être utilisée dans le traitement d'une maladie ou d'un trouble fortement associé à la signalisation de JNK chez un sujet par une administration intravésicale, dans lequel la maladie ou le trouble fortement associé à la signalisation de JNK chez un sujet est une cystite, en particulier une cystite interstitielle, et dans lequel le peptide chimérique est constitué de la séquence d'acide aminé SEQ ID NO: 11.

2. Peptide chimérique selon la revendication 1, dans lequel la séquence inhibitrice de JNK se lie à la kinase amino terminale c-jun (JNK).

3. Peptide chimérique destiné à être utilisé selon l'une ou l'autre des revendications 1 et 2, dans lequel la séquence inhibitrice de JNK inhibe l'activation d'au moins un facteur de transcription ciblé par JNK lorsque la séquence inhibitrice de JNK est présente dans une cellule exprimant JNK.

4. Peptide chimérique destiné à être utilisé selon l'une quelconque des revendications 1 à 3, dans lequel le facteur de transcription ciblé par JNK est sélectionné dans le groupe constitué de c-jun, d'ATF2 et d'Elk1.

5. Peptide chimérique destiné à être utilisé selon l'une quelconque des revendications 1 à 4, dans lequel la séquence inhibitrice de JNK altère un effet de JNK lorsque le peptide est présent dans une cellule exprimant JNK.

6. Peptide chimérique destiné à être utilisé selon l'une quelconque des revendications 1 à 5, dans lequel les séquences de trafic augmentent l'absorption cellulaire du peptide.

7. Peptide chimérique destiné à être utilisé selon l'une quelconque des revendications 1 à 6, dans lequel la séquence de trafic dirige une localisation nucléaire du peptide.
